(19) Europäisches Patentamt
European Patent Office
Office européen des brevets

(11) **EP 4 597 514 A1**

(12) **EUROPEAN PATENT APPLICATION**
published in accordance with Art. 153(4) EPC

(43) Date of publication:
06.08.2025 Bulletin 2025/32

(21) Application number: 23872464.5

(22) Date of filing: 27.09.2023

(51) International Patent Classification (IPC):
*G16H 50/00* (2018.01)     *G06N 20/00* (2019.01)
*G16H 10/40* (2018.01)

(52) Cooperative Patent Classification (CPC):
**G06N 20/00; G16H 10/40; G16H 50/00**

(86) International application number:
**PCT/JP2023/035267**

(87) International publication number:
**WO 2024/071242 (04.04.2024 Gazette 2024/14)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: 28.09.2022 JP 2022155487
26.05.2023 JP 2023086586

(71) Applicant: **RIKEN**
**Wako-shi, Saitama 351-0198 (JP)**

(72) Inventor: **YAMAMOTO, Yoichiro**
**Wako-shi, Saitama 351-0198 (JP)**

(74) Representative: **Epping - Hermann - Fischer
Patentanwaltsgesellschaft mbH
Schloßschmidstraße 5
80639 München (DE)**

(54) **SUGGESTION DEVICE, SUGGESTION METHOD, SUGGESTION SYSTEM, PROGRAM, AND INFORMATION RECORDING MEDIUM FOR SUGGESTING FACTOR PARAMETERS FOR ESTIMATING TARGET LABEL**

(57) The present disclosure suggests a factor parameter for estimating a label of a target from a plurality of parameters that can be acquired for the target. In a suggestion device (101), a calculator (102) learns, by referring to a plurality of records in which each record represents a plurality of parameter values acquired from the target and a label provided to the target, an estimation model that estimates a label from a parameter related to a combination for a part of calculation candidates among a plurality of candidates representing a combination of parameters; calculates performance of the estimation model; causes calculation candidates to compete based on the calculated performance; and calculates a rating value of the calculation candidate. An estimator (103) learns a rating model by a rating value and a combination of parameters of the calculation candidate, and estimates a rating value of an estimation candidate other than the calculation candidate. An outputter (104) outputs, as a useful candidate representing a factor parameter, a candidate having a calculated or estimated high-ranking rating value and being the calculation candidate.

FIG. 1

## Description

Technical Field

The present disclosure relates to a suggestion device, a suggestion method, a suggestion system, a program, and an information recording medium for suggesting a factor parameter for estimating a label of a target among a plurality of parameters that can be acquired for the target.

Background Art

A degree of pathological malignancy of prostate cancer is an important evaluation value to decide a treatment plan. Whether a disease is pathologically malignant and a degree of pathological malignancy can be checked by performing, by using a microscope, a pathological examination of a sample acquired from a patient. The inventor proposes a technique for acquiring a feature parameter or a feature vector by performing dimensionality compression on a photograph image acquired by capturing a sample acquired from a target being a patient, and then appropriately classifying the target being the patient by whether a disease is pathologically malignant, based on the acquired feature parameter or the acquired feature vector (Patent Literature 1).

However, a pathological examination is not only invasive but also requires time until an examination result comes out and a drug price is also high. Therefore, the pathological examination is greatly useful for a post-analysis after a removal operation of an executive but is difficult to apply to a preliminary prediction.

Thus, there is a request to improve accuracy in predicting a prognosis and a treatment effect by appropriately combining and using capturing of a prostate cancer echo image, various noninvasive examinations such as a blood examination, and the like, by predicting a degree of whether a disease is pathologically malignant in advance, and by further combining information acquired from an invasive examination such as information based on a photograph image in which a sample created from an affected area cut by a treatment operation is captured.

Herein, due to development of medical science, kinds of examinations starting with blood examinations are increasing steadily. In order to perform various examinations, effort, time, a cost based on a drug price, and the like are required. Thus, a combination of examinations having good cost performance, that is, a combination of examinations greatly contributing to appropriate estimation of a current state of a disease of a patient, a prognosis, a treatment effect, and the like, needs to be selected.

This problem can be generalized as follows. In other words, a technique for specifying which factor parameter contributes to a decision of a label of a target among a plurality of parameters that can be acquired for the target, and a combination of the factor parameters is desired.

When there are n kinds of parameters that can be acquired from a target, a combination of the parameters is $2^n$ kinds in total. Therefore, in this technique, a candidate representing a suitable combination of factor parameters to estimate a label is specified from $2^n$ candidates each representing a possible combination of the parameters. Herein, the suitable combination of the factor parameters is useful to estimate the label of the target, and thus a candidate representing the suitable combination can be referred to as a useful candidate.

Herein, in order to narrow the $2^n$ candidates down to the useful candidate, a conceivable technique is to obtain superiority or inferiority by performing a competition for comparing performance between the candidates by using a rating system such as Glicko Rating and Glicko 2 Rating in addition to Elo Rating and TrueSkill (trademark) disclosed in Patent Literature 2, to calculate a rating value of each of the candidates, based on the superiority or inferiority, and then to set a candidate having a high-ranking rating value as the useful candidate.

Citation List

Patent Literature

Patent Literature 1: Japanese Patent No. 6945253
Patent Literature 2: United States Patent No. 8538910

Summary of Invention

Technical Problem

However, in order to calculate performance of a candidate representing a combination of parameters, learning of an estimation model needs to be advanced by a technique such as machine learning, a neural network, and deep learning in which a parameter value of a parameter related to the combination among parameters that can be acquired from a target is used as input data, and a label provided to the target is used as output data. Learning of the estimation model often requires a great calculation amount and a long time.

Meanwhile, many candidates need to be caused to compete in the rating system. Most simply, the estimation model is obtained for all candidates, performance of the estimation model is calculated, and a competition between the candidates is then performed under the rating system. However, in this technique, both of a calculation amount and a calculation time are enormous, and an explosion in a competition number occurs.

Therefore, a technique for appropriately narrowing candidates down to a useful candidate with a small calculation amount in a short calculation time while suppressing learning of the estimation model and the number of competitions, and presenting the acquired useful candidate is desired.

The present disclosure is to solve the problems described above, and has an objective to provide a suggestion device, a suggestion method, a suggestion system, a program, and an information recording medium for suggesting a factor parameter for estimating a label of a target among a plurality of parameters that can be acquired for the target.

Solution to Problem

A suggestion device according to the present disclosure suggests, based on a plurality of records, any one or more candidates of a plurality of candidates each representing a combination of parameters to be acquired for a target, as a useful candidate representing a combination of factor parameters contributing to a decision of a label provided to the target. Herein, each record of the plurality records includes a plurality of parameter values each acquired by acquiring a plurality of parameters for a target associated with the each record, and a label provided to the associated target. Then, the suggestion device

learns, by referring to the plurality of records, an estimation model for each calculation candidate of a calculation candidate being a part of the plurality of candidates,

calculates, by the learned estimation model, performance that estimates a label provided to a target from a parameter value in which a parameter related to a combination represented by the each calculation candidate is acquired for the target,

calculates a rating value associated with the each calculation candidate, based on the calculated performance,

learns a rating model by referring to a rating value calculated for the calculation candidate,

estimates a rating value associated with each estimation candidate of an estimation candidate other than the calculation candidate among the plurality of candidates, based on the learned rating model, and

outputs, as the useful candidate, a calculation candidate associated with a rating value being high-ranking among the calculated or estimated rating values.

A suggestion system according to the present embodiment is a prediction system for predicting a prognosis of a patient for a medical worker who deals with the patient, and the suggestion system

presents an already-acquired examination value for the patient on a screen,

selects, as a prediction candidate, a useful candidate in which the already-acquired examination value fulfills a combination of examination items related to a useful candidate, from a plurality of useful candidates being a plurality of useful candidates and each representing a combination of examination items,

provides, as an input, the already-acquired examination value that fulfills a combination related to the selected prediction candidate to an estimation model learned for the selected prediction candidate, and causes the estimation model to estimate a prognosis of the patient, and

presents the estimated prognosis on the screen.

Advantageous Effects of Invention

The present disclosure can provide a suggestion device, a suggestion method, a suggestion system, a program, and an information recording medium for suggesting a factor parameter for estimating a label of a target among a plurality of parameters that can be acquired for the target.

Brief Description of Drawings

FIG. 1 is an explanatory diagram illustrating an overview configuration of a suggestion device according to an embodiment of the present disclosure;

FIG. 2 is an explanatory diagram illustrating a network configuration of deep learning used for a rating model;

FIG. 3 is a flowchart illustrating a basic flow of control of suggestion processing according to the embodiment of the present disclosure;

FIG. 4 is a flowchart illustrating a flow of the control in a modified example of the suggestion processing according to the embodiment of the present disclosure;

FIG. 5 is an explanatory diagram illustrating an output example of outputting a useful candidate by a scatter diagram;

FIG. 6 is an explanatory diagram illustrating a display example of outputting a useful candidate in the scatter diagram and displaying the useful candidate on a screen;

FIG. 7 is an explanatory diagram illustrating a display example of outputting a useful candidate in the scatter diagram and displaying the useful candidate on a screen;

FIG. 8 is an explanatory diagram illustrating an overview configuration of a suggestion system according to the embodiment of the present disclosure;

FIG. 9 is an explanatory diagram illustrating a scene of transmission and reception of information in the suggestion system according to the embodiment of the present disclosure;

FIG. 10 is an explanatory diagram illustrating a scene of transmission and reception of information in the suggestion system according to the embodiment of the present disclosure;

FIG. 11 is an explanatory diagram illustrating an overview configuration of a prediction system included in the suggestion system according to the embodiment of the present disclosure;

FIG. 12 is an explanatory diagram illustrating a scene of an electronic medical chart presented in the prediction system according to the embodiment of the present disclosure;

FIG. 13 is an explanatory diagram illustrating a scene where a result of a prognosis prediction is presented in the electronic medical chart presented in the prediction system according to the embodiment of the present disclosure; and

FIG. 14 is a flowchart illustrating a flow of control of prediction processing executed in the prediction system according to the embodiment of the present disclosure.

Description of Embodiments

Embodiments according to the present disclosure are described below. Note that the present embodiment is used for description, and does not limit the scope of the present disclosure. Therefore, a person skilled in the art can adopt an embodiment in which each element or all elements of the present embodiment are replaced with equivalent elements. Further, an element described in each example can also be appropriately omitted according to a use. In this way, all embodiments constituted based on the principle of the present disclosure are included in the scope of the present disclosure.

Configuration

A suggestion device according to the present embodiment is typically achieved by a computer executing a program. The computer is connected to various output devices and input devices, and transmits and receives information to and from the devices.

The program executed by the computer can be distributed and sold by a server communicably connected to the computer. In addition, the program can be recorded in a non-transitory information recording medium such as a compact disk read only memory (CD-ROM), a flash memory, and an electrically erasable programmable ROM (EEPROM), and then the information recording medium can be distributed or sold.

The program is installed in the non-transitory information recording medium such as a hard disk, a solid state drive, a flash memory, and an EEPROM included in the computer. Then, the computer achieves an information processing device according to the present embodiment. In general, a central processing unit (CPU) of the computer reads the program from the information recording medium into a random access memory (RAM) under management by an operating system (OS) of the computer, and then interprets and executes a code included in the program. However, in an architecture in which the information recording medium can be mapped in a memory space accessible by the CPU, explicit loading of the program into the RAM may be unnecessary. Note that various types of information needed in a process of execution of the program may be temporarily recorded in the RAM.

Furthermore, as described above, the computer desirably includes a graphics processing unit (GPU), and includes the GPU for performing various image processing calculations at a high speed. By using the GPU and a library such as TensorFlow, a learning function, a suggestion function, a dimensionality compression function, and the like in various types of artificial intelligence processing, such as a neural network, machine learning, and deep learning, can be used under control of the CPU.

Note that, instead of achieving the information processing device according to the present embodiment by a general-purpose computer, the information processing device according to the present embodiment can also be constituted by using a dedicated electronic circuit. In this aspect, the program can also be used as a material for generating a wiring drawing, a timing chart, and the like of the electronic circuit. In such an aspect, the electronic circuit that satisfies specifications determined in the program is constituted by a field programmable gate array (FPGA) and an application specific integrated circuit (ASIC), the electronic circuit functions as a dedicated apparatus that achieves a function determined in the program, and achieves the information processing device according to the present embodiment.

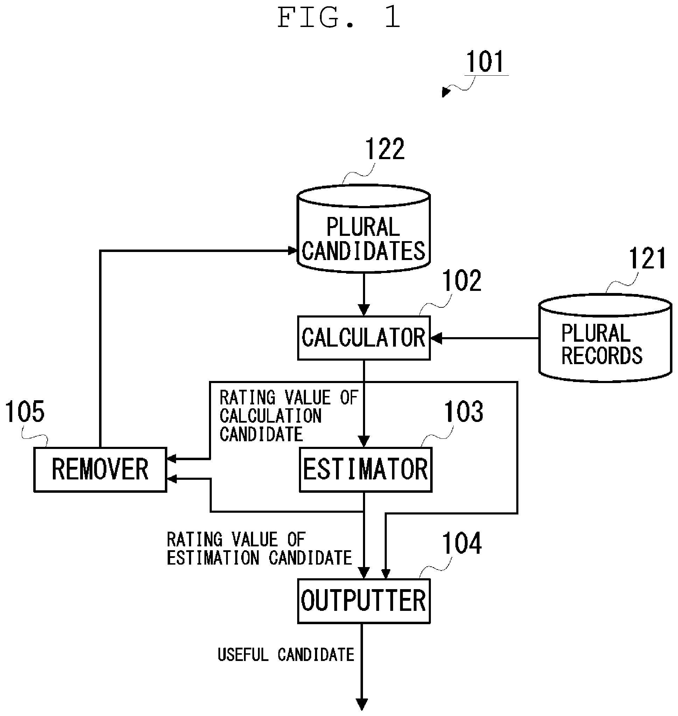

Hereinafter, in order to facilitate understanding, description is given by assuming an aspect in which the suggestion device is achieved by a computer executing a program. FIG. 1 is an explanatory diagram illustrating an overview configuration of the suggestion device according to the embodiment of the present disclosure.

As illustrated in FIG. 1, a suggestion device 101 according to the present embodiment includes a calculator 102, an estimator 103, and an outputter 104. Further, a remover 105 can be provided as an omittable element.

The suggestion device 101 suggests, based on a plurality of records, any one or more candidates of a plurality of candidates each representing a combination of parameters that can be acquired for a target, as a useful candidate representing a combination of factor parameters contributing to a decision of a label provided to the target.

As described above, when an n kind of a parameter can be acquired for a target, a total number of combinations of the parameters is $2^n$, and thus each of the combinations can be expressed by an n-dimensional binary vector. For example, expressions can be made in such a way that an i-th parameter is included in a combination when a value of an i-th element of a binary vector is 1, and the i-th parameter is not included in the combination when a value of the i-th element is 0.

For example, when 13 kinds of examinations of PSA, WBC, Hb, Plt, LDH, ALP, GOT, GPT, Alb, Bun, cre, Ca, and CRP are assumed as blood examinations, n = 13. A combination can be expressed by 13-dimensional binary vectors that represent whether each examination is to be included or not included in the combination by 1 or 0 and are arranged in order.

For example, a combination in which an examination is performed by adopting parameters that acquire three examinations of PSA (tumor marker), WBC (white blood cell count), and Hb (hemoglobin), and an examination is not performed by not considering the other parameter as an acquisition target is expressed by 13-dimensional binary vectors of 1, 1, 1, 0, 0, 0, 0, 0, 0, 0, 0, 0, 0].

Note that, as an estimation model for estimating, from a parameter value acquired from a target, a label that needs to be provided to the target, various machine learning models and classification models such as Support Vector Machine (SVM), Lasso, Ridge, Elasticnet, Decision Tree, Random Forest (RF), CART, Boosting, XGGoost, and various neural networks can be adopted. Further, the various models can also be used in combination.

In the classification models, various hyperparameters need to be set in order to adjust behavior of the classification models. In other words, behavior of the classification models changes according to setting of a specification.

Thus, a classification and a specification of the estimation model may be associated with each candidate.

For example, when 10 kinds described above are assumed as the estimation models for the blood examinations described above and each of 5 kinds is assumed as a specification of each of the estimation models, the number of candidates is $2^{13} \times 10 \times 5$ in total. Further, a certain parameter can also be always included in a combination. For example, when PSA is always included, the number of candidates is $2^{(13-1)} \times 10 \times 5$, which results in a half of the number described above.

Note that, when only one kind is adopted as the estimation model, a classification and a specification of the model do not need to be represented by each candidate.

The plurality of candidates is generated and stored in a candidate storage area 122 such as a RAM by listing combinations.

As described above, the suggestion device 101 according to the present embodiment refers to a plurality of records being recorded in a database 121 and the like.

For example, in a case of a prostate cancer patient described above, each record corresponds to an electronic medical chart including results of the 13 kinds of the blood examinations that have been performed on the prostate cancer patient.

This electronic medical chart may include various medical images (such as a pathological photograph image acquired by an invasive examination including a prostate biopsy and the like, an echo photograph image acquired by an ultrasonic examination, CT, MRI, PET, skeleton sintigraphy, a simple X ray, mammography, dermoscopy, a fundus photograph, and a corneal photograph), a feature parameter or a feature vector acquired by performing dimensionality compression on the medical images by the technique disclosed in Patent Literature 1 and the like, and the like.

The electronic medical chart may also include a Gleason score determined by observing, by a doctor and the like, a pathological sample (or a pathological photograph image) acquired by an invasive examination being a prostate biopsy, and a determination result of whether a tumor is malignant, based on the Gleason score.

Hereinafter, in order to facilitate understanding, description is given on an assumption that, as one example, an electronic medical chart includes a Gleason score determined by observing, by a doctor and the like, a pathological sample acquired by an invasive examination such as a prostate biopsy, and (a feature vector and a feature parameter of) an echo photograph image serving as a medical image, but an application range of the present example is not limited to this.

Furthermore, the electronic medical chart usually includes a patient's gender, age, medical history, presence or absence and frequency of drinking and smoking, an examination result other than a blood examination, and the like as well.

The information generally corresponds to a parameter value of a parameter acquired for a target.

Furthermore, the electronic medical chart also includes information about a prognosis of the patient after a concerned examination, presence or absence of a recurrence, and the like. The information generally corresponds to a label provided to a target.

In other words, each record of a plurality records includes a plurality of parameter values each acquired by acquiring a plurality of parameters for a target associated with each of the records, and a label provided to the associated target.

The calculator 102 of the suggestion device 101 refers to the plurality of records, for example, a database of the electronic medical chart described above, and executes the following processing.

In other words, the calculator 102 refers to the plurality of candidates generated in the candidate storage area 122 and the like, and learns the estimation model for each calculation candidate of a calculation candidate being a part of the plurality of candidates.

As described above, a combination of parameters that need to be acquired from a plurality of parameters is associated with each candidate. As described above, a kind and a specification of the estimation model that performs learning by using the combination are also generally associated with each candidate.

The calculator 102 learns, for a part of the plurality of candidates, the estimation model of a kind and a specification indicated by the candidate. In other words, in the present specification, a candidate in which the estimation model associated with the candidate is learned is referred to as a "calculation candidate".

The estimation model related to each calculation candidate sets, as an input, a parameter value in which a parameter related to a combination represented by each calculation candidate for a target is acquired, and sets the target as an output.

For example, in a case of a candidate related to a combination expressed by the 13-dimensional binary vectors of [1, 1, 1, 0, 0, 0, 0, 0, 0, 0, 0, 0, 0] described above, an input of the estimation model is examination results of the three blood examinations of PSA, WBC, and Hb.

Note that, as an input other than a parameter related to a combination indicated by a candidate, a feature parameter or a feature vector acquired by performing dimensionality compression on an image in which a target is captured can also be adopted. When this aspect is considered in the example described above, a vector including the examination results of the three blood examinations of PSA, WBC, and Hb, and the feature parameter or the feature vector acquired by performing the dimensionality compression and the dimensionality compression on the echo photograph image is input data for the estimation model.

Furthermore, information that can be easily acquired from a patient without requiring an examination, such as gender, age, medical history, and presence or absence and frequency of drinking and smoking, can also be adopted as input data.

An output of the estimation model is (probability of) a label that needs to be provided to a target related to input data. The estimation model compares an evaluation value calculated based on the input data with a threshold value, and performs classification. Then, by learning the estimation model, various coefficients used for calculation of the evaluation value (and a threshold value to be compared, depending on a classification of the model) are determined.

When a label is expressed by binary expression of negative and positive, an evaluation value calculated by the estimation model can be constituted in such a way as to represent a probability that a target related to input data is classified into one (for example, positive) of positive and negative. In this case, a threshold value is a constant of 0.5.

A plurality of records is referred for learning of the estimation model. The plurality of records is divided into a training record and a test record. The remainder acquired by removing the training record from the plurality of records is the test record. The division may be randomly performed, or may be regularly performed based on a predetermined rule and a predetermined procedure.

Training data for learning the estimation model are generated from the training record. Input data in each piece of the training data includes a parameter value in which a parameter related to a combination represented by each candidate is extracted from each training record. The parameter value is referred to as a combination input parameter. In the example described above, the combination input parameter corresponds to an examination value of the blood examinations of PSA, WBC, and Hb related to the electronic medical chart of the patient.

Further, information other than a combination of parameters can also be added as the input data. The information is referred to as an additional input parameter. In the example described above, the additional input parameter is gender, age, medical history, presence or absence and frequency of drinking and smoking, and the like.

Note that, when a prognosis and an effect of an operation after an invasive operation has been performed on a patient are desired to be predicted, a Gleason score determined by observing, by a doctor and the like, a pathological sample acquired by the operation, and a feature parameter and a feature vector acquired by performing dimensionality compression on the pathological photograph image can be adopted as the input data. The input data can be applied to both of the combination input parameter and the additional input parameter.

Output data (correct data) are a label representing a prognosis of the patient and an effect of an operation that are indicated in the training record. In the example described above, the label represents whether prostate cancer has recurred within a predetermined period (for example, a year) after an operation, and, for example, a value such as 1 in a case of a recurrence and 0 in a case of no recurrence is assigned.

In this way, the training data according to each candidate are generated from the training record, and the estimation model is learned.

When learning of the estimation model related to each calculation candidate ends, the calculator 102 calculates performance in which the learned estimation model estimates the label provided to the target from the parameter value in which the parameter related to the combination represented by each calculation candidate for the target is acquired.

Test data generated from the test record in a similar procedure to that of the training record are used for the calculation of the performance.

In other words, each piece of the test data is subjected to a test of whether both of the label output from the estimation model and the output data related to the test data coincide with each other by providing the input data to the learned estimation model. When both of the label and the output data coincide with each other, an inference by the estimation model is correct. Most simply, a correct rate acquired herein can be set as performance of the learned estimation model.

A label of an estimation result and a probability related to the label may be included in an output from the estimation model. In this case, the calculator 102 can calculate an area under curve (AUC) value by plotting a receiver operating characteristic (ROC) curve, based on a result of each test being performed. Then, the calculator 102 can set the calculated AUC value as performance of the learned estimation model.

Note that, for a record in which a part of parameters is not acquired, for example, an electronic medical chart of a patient on which only a part of blood examinations has been performed, when a parameter value related to combination input data and information related to additional input data are available in the record, the record can also be adopted as the training record or the test record.

In this way, when performance of the learned estimation model related to the calculation candidate is calculated, the calculator 102 calculates a rating value associated with the calculation candidate, based on the calculated performance.

The rating value according to the present embodiment is similar to that in Elo Rating and the like, and is used for acquiring a winning probability from a difference in the rating value between competitors.

In the present embodiment, by referring to the performance calculated for the calculation candidate among the plurality of candidates, the calculation candidates are caused to compete against each other, and rating values of the calculation candidates that have competed are updated in such a way that the rating value of the calculation candidate that has won increases and the rating value of the calculation candidate that has lost decreases, based on winning and losing of the competitions.

For example, in Elo Rating, a rating value of an average competitor, that is, an initial value of a rating value before update is a constant. As the initial value, for example, 1500 can be used.

In Elo Rating, a percentage of winning of a competitor is estimated based on a rating value before a competition. For example, when rating values of competitors a and b are Ra and Rb, probabilities of winning Wab and Wba of each of a and b are estimated as

$$\mathrm{Wab} = 1/[10^{(Rb - Ra)/400} + 1],$$

and

$$\mathrm{Wba} = 1/[10^{(Ra - Rb)/400} + 1] = 1 - \mathrm{Wab}.$$

Herein, a constant of 400 is used, but the other value can also be adopted.

Then, when the competitors compete against each other for several times, the rating values are updated according to a disparity between an estimated percentage of winning and an actual percentage of winning. When an actual percentage of winning of the competitor a is Sab and an actual percentage of winning of the competitor b is Sba = 1 - Sab after the competitors a and b have competed against each other for several times, new rating values Ra' and Rb' of the competitors a and b are updated in such a way that

$$Ra' = Ra + K \times (Sab - Wab),$$

and

$$Rb' = Rb + K \times (Sba - Wba).$$

Herein, 16 and 32 are often used as a value of K.

In this way, the calculator 102 obtains winning and losing of competitions between calculation candidates that have competed, based on performance of the calculation candidates, and calculates rating values of the calculation candidates by updating the rating values associated with the calculation candidates that have competed.

In order to keep a fair competition between the calculation candidates, the training data and the test data provided to both of the calculation candidates need to be common.

Thus, the calculator 102 extracts two calculation candidates from among a plurality of candidates. The extraction of the two calculation candidates is typically randomly performed, but other various ways are described below.

Then, the calculator 102 repeats the following processing for several times for the two extracted calculation candidates. A way of deciding the number of times of repetition, that is, the number of times the same calculation candidates compete against each other is described below.

(a) A training record is randomly selected from a plurality of records, and a remaining record is set as a test record,
(b) training data and test data are generated,
(c) an estimation model is learned by the training data,
(d) each piece of performance of the two calculation candidates is calculated based on the two learned estimation models, and
(e) winning and losing of a competition this time between the two calculation candidates are decided by a level of the performance.

Then, the two calculation candidates compete against each other for one or more times, a probability of winning and losing of the competition between the two calculation candidates is obtained. Thus, rating values of both of the calculation candidates are updated by setting the calculation candidate having a higher percentage of winning as a winner and setting the calculation candidate having a lower percentage of winning as a loser. As the number of times of competitions repeated by the two calculation candidates, most simply, one or more constants may be adopted, but can be appropriately determined.

In the processing (a), when the same two calculation candidates repeat a competition for two or more times, the training data and the test data are randomly selected at the beginning of each of the competitions, and thus the training data and the test data are different between a certain competition and another competition. Therefore, which candidate wins may change for each competition.

In the processing (c), when the estimation model has already been learned in a previous competition, a learning result up to the present may be carried on, but the estimation model may be cleared first, and then learning by the training data this time may be performed.

When rating systems other than Elo Rating, for example, Glicko Rating and Glicko 2 Rating are adopted, similar processing may also be executed. A rating system such as TrueSkill (trademark) supports a competition among three or more competitors. In such a rating system, the calculator 102 may extract three or more candidates, cause the candidates to compete against one another, and update rating values.

The calculator 102 repeats, for several times, processing of "selecting calculation candidates, repeating a competition for several times, obtaining a percentage of winning of both of the calculation candidates, based on competition results, and then updating rating values of both of the calculation candidates". The number of times of repetition, that is, the number of times the calculation candidates are extracted can be set to, for example, a constant, but the other technique is described below.

As described above, a plurality of candidates has an enormous number according to a combination of parameters and a classification and a specification of an estimation model, and thus it is difficult to cause all combinations of the candidates to compete against one another.

Thus, after a competition proceeds to some extent, the calculator 102 refers to a rating value calculated for a calculation candidate, and learns a rating model. Note that, at a time of the learning, in order to avoid a local solution, correction may be performed in such a way as to change a part of rating values, and the rating values after the correction may be referred. The calculation candidate whose rating value is to be changed can be selected from the whole of the calculation candidates, for example, randomly. For a change amount of the rating value, techniques such as addition, subtraction, multiplication, and division of a constant value or a random value are conceivable.

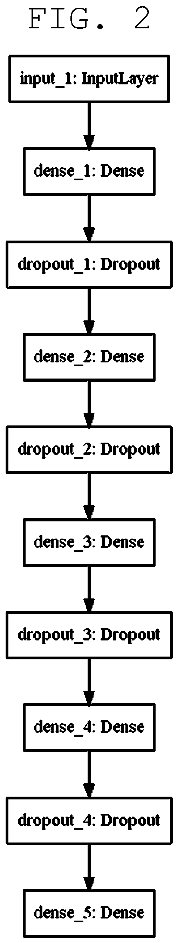

Various classifications and specifications of rating models can be adopted similarly to the estimation model. FIG. 2 is an explanatory diagram illustrating a network configuration of deep learning used for a rating model. The network configuration illustrated in FIG. 2 adopts a simple fully-connected configuration in a deep learning model.

In training data for the rating model, input data are a combination of parameters (and a classification and a specification of the estimation model) indicated by a candidate, and output data (correct data) are a rating value of the candidate.

In the example described above, the input data are 13-dimensional binary vectors (and the classification and the specification of the estimation model) related to a calculation candidate.

A candidate that has not been extracted as a calculation candidate and has not thus competed even one time and has not been subjected to calculation of a rating value (update from an initial value) is referred to as an estimation candidate in the present embodiment. When a combination of parameters (and the classification and the specification of the estimation model) indicated by the estimation candidate are provided as an input to a learned rating model, the rating model estimates a rating value of the estimation candidate, and outputs the estimated rating value.

Candidates whose combinations of parameters are similar also often have similar rating values, and the rating model conceivably learns aspects of "similarity between candidates" and "similarity between rating values" herein.

Then, the calculator 102 estimates a rating value associated with each of the estimation candidates, based on the learned rating model.

In this way, a candidate having an estimation model learned through a competition and having a rating value calculated is classified as a calculation candidate, and a candidate having no competition yet and having an estimation model not learned has a rating value estimated by the rating model and is classified as an estimation candidate.

Then, the outputter 104 outputs, as a useful candidate, the calculation candidate associated with a high-ranking rating value among the calculated or estimated rating values.

For example, the outputter 104 selects a predetermined number of candidates (hereinafter referred to as "high-ranking candidates") having an associated high-ranking rating value from a plurality of candidates. When the high-ranking candidates are all calculation candidates, the outputter 104 outputs the high-ranking candidates as useful candidates.

At this time, the outputter 104 can further output an estimation model learned for the useful candidate. For example, when a doctor selects an examination that needs to be performed on a patient, combinations of examinations suggested by output useful candidates are compared and discussed, and, after any of the combinations is selected and the examination is performed, a prognosis and the like of the patient can be predicted by providing an examination value to an estimation model related to the combination. The embodiment for comparing and contrasting output useful candidates is described below.

The outputter 104 can also make it easy for a person to determine whether any of a plurality of useful candidates needs to be adopted, by illustrating the useful candidates in a scatter diagram.

Herein, a first axis (for example, a vertical axis) in the scatter diagram can indicate the number of parameters related to a combination represented by a useful candidate, or a total cost for acquiring a parameter related to the combination (for example, a drug price when an examination related to a parameter is performed), and a second axis (for example, a horizontal axis) in the scatter diagram can indicate performance (for example, an AUC value) or a rating value calculated for the useful candidate.

When the horizontal-vertical setting described above is adopted, the useful candidate located on the lower side in the vertical axis (the number of parameters or the total cost is small) and on the right side in the horizontal axis (the performance or the rating value is high) is desirable.

When a part or a whole of high-ranking candidates is an estimation candidate, the estimation model is not learned for the estimation candidate, and thus the processing described above needs to be repeated.

Most simply, the processing of extracting an estimation candidate being a high-ranking candidate as a calculation candidate, and performing calculation by the calculator 102 and estimation by the estimator 103 may be repeated, and an output by the outputter 104 may be then performed.

Most simply, in an aspect in which a candidate to be caused to compete is randomly extracted every time, all of high-ranking candidates become calculation candidates at some point by advancing the whole repetition. However, in order to reduce a competition having a small benefit, the remover 105 may remove an estimation candidate having an associated low-ranking rating value from the candidate storage area 122 and the like, and narrow down candidates at an appropriate timing, for example, a timing at which it becomes clear that a part or a whole of high-ranking candidates is an estimation candidate and the processing thus needs to be repeated one more time.

In step S205, the calculator 102 may perform random extraction from a plurality of candidates while prioritizing a candidate that has not learned an estimation model for the candidate or a candidate having a small number of times of learning, and thus the extracted candidate may be set as a calculation candidate for a subsequent competition. In other words, a probability that a candidate is extracted may be changed according to presence or absence and the number of times of learning of the estimation model. Alternatively, a technique for not subsequently extracting a candidate that has learned the estimation model or has a sufficiently great number of times of learning may be used.

Basic Flow

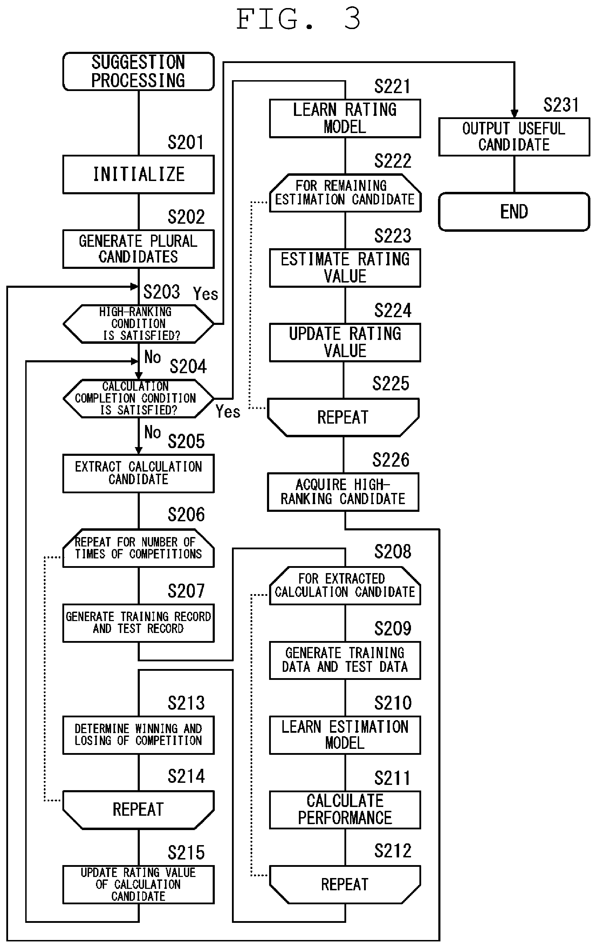

A basic flow of control according to the present embodiment is described below. FIG. 3 is a flowchart illustrating a basic flow of control of suggestion processing according to the embodiment of the present disclosure.

When the processing starts, the suggestion device 101 according to the present embodiment performs various types of initialization such as acquisition of a plurality of records from the database 121, setting of a plurality of parameters that can be acquired from a target, and setting of a classification and a specification of an adoptable estimation model (step S201).

Next, the suggestion device 101 generates a plurality of candidates by listing a combination of the plurality of set parameters and the set classification and the set specification of the estimation model, and stores, in the candidate storage area 122, the plurality of candidates in association with a rating value to which a default value is set (step S202).

Subsequently, when a high-ranking condition is not satisfied (step S203; No), the suggestion device 101 repeats the following processing. As the high-ranking condition, for example, a condition that all of candidates having a high-ranking rating value are calculation candidates can be adopted. When step S203 is reached for the first time, it is likely that no calculation candidate is present and rating values of all candidates are the same initial value, and thus the high-ranking condition is not satisfied, and the control proceeds to step S204.

Next, when a calculation completion condition is not satisfied (step S204; No), the suggestion device 101 repeats the following processing. As the calculation completion condition, a condition that learning of an estimation model, calculation of performance, and calculation of a rating value are completed for the number of calculation candidates to some extent can be adopted, and a simplest condition that repetition is performed for a predetermined number of times can be adopted. When step S203 is reached for the first time, no calculation candidate is present, and thus the calculation completion condition is not satisfied, and the control proceeds to step S205.

First, in the suggestion device 101, the calculator 102 extracts a calculation candidate from the plurality of candidates stored in the candidate storage area (step S205). The number of the calculation candidates extracted herein is the number of competitors that can take part in one competition in an adopted rating system, and is, for example, 2 in Elo Rating.

Then, the calculator 102 repeats the following processing for only a predetermined number of competitions (step S206). The number of competitions may be set to a fixed number of one or more times, or may be appropriately changed.

First, the calculator 102 randomly divides a plurality of records, and generates a training record and a test record (step S207). The number of the training records is generally set several times to several tens of times as large as the number of the test records.

Then, the calculator 102 repeats the following processing for each of the calculation candidates extracted in previous step S206 (step S208).

First, the calculator 102 generates training data and test data from the training record and the test record, based on a combination represented by the calculation candidate (step S209).

Next, the calculator 102 provides the generated training record to an estimation model related to the classification and the specification represented by the calculation candidate, and learns the estimation model (step S210). Note that, when the estimation model has already been learned in the past repetition, the estimation model may be cleared, or additional learning may be performed without clearing the estimation model.

After the estimation model is learned, the calculator 102 provides the generated test data to the learned estimation model, and calculates performance of the estimation model (step S211).

When the performance of the estimation model is calculated for each of the calculation candidates extracted by repetition (step S212), the calculator 102 determines winning and losing of a competition between the extracted calculation candidates, based on the calculated performance (step S213).

When a predetermined number of times of the competitions is performed by repetition (step S214), the calculator 102 updates a rating value stored in association with each of the extracted calculation candidates in the candidate storage area, based on the number of winning and losing in the predetermined number of times of the competitions (step S215), and the control returns to step S204. The updated rating value is a newest calculated rating value for the calculation candidate.

In this way, in the present embodiment, the candidate extracted in step S206 is set as the calculation candidate, and learning of an estimation model, calculation of performance, and calculation of a rating value are performed through the processing in steps S207 to S215.

When the calculation completion condition is satisfied (step S204; Yes), the estimator 103 learns a rating model by using, as the training data, a combination of parameters (and a classification and a specification of an estimation model) represented by the calculation candidate, and a rating value calculated for the calculation candidate (step S221). When the whole repetition has been performed, the rating model that has been learned up to a previous time may be used as it is and additional learning may be performed, or the rating model may be cleared and then learning may be performed.

Then, the following processing is repeated for each of remaining candidates not being the calculation candidates, that is, estimation candidates among the plurality of candidates (step S222).

First, the combination of the parameters (and the classification and the specification of the estimation model) represented by the estimation candidate are provided as an input to the learned rating model, and a rating value is estimated (step S223).

Next, the rating value stored in association with the estimation candidate in the candidate storage area is updated by the estimated rating value (step S224).

Whether the candidate is the calculation candidate or the estimation candidate, that is, whether the rating value stored in the candidate storage area is the calculated value or the estimated value can be identified by whether the estimation model for the candidate has already been learned.

When estimation of the rating values for all of the estimation candidates ends (step S225), the suggestion device 101 acquires a high-ranking candidate having a high-ranking rating value from the candidates stored in the candidate storage area (step S226), and the control returns to step S203. Herein, most simply, a predetermined number of the candidates from the top when the candidates are arranged in a descending order of the rating value can be set as high-ranking candidates. In addition, as the definition of a "rating value being high-ranking", the number of parameters included in a combination of each candidate, an added score by assigning weight to a parameter included in a combination of each candidate (for example, a drug price when an examination related to a combination is performed), and the like may be taken into consideration.

When the high-ranking condition is satisfied (step S203; Yes), the outputter 104 outputs a useful candidate, based on the acquired high-ranking candidate (step S231), and the present processing ends.

Most simply, the outputter 104 outputs the high-ranking candidate as the useful candidate as it is.

In addition, instead of setting all of the high-ranking candidates as the useful candidates, a rating value may be further updated by performing a competition between the high-ranking candidates in a round-robin manner, and then a part of the high-ranking candidates having a high rating value after the round-robin competition may be output as the useful candidate.

The basic flow of the control according to the present embodiment is described above, and an example acquired by modifying the basic flow of the control is described below.

Modified Flow

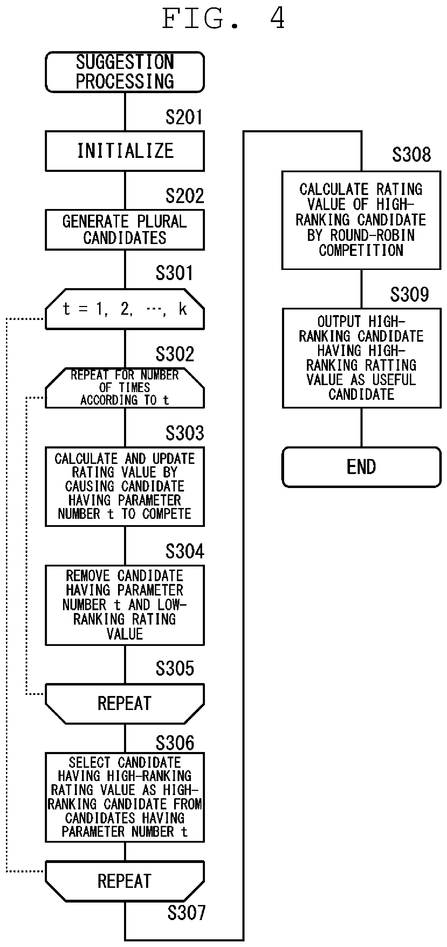

A modified example in which an upper limit k is provided for the number of parameters represented by a useful candidate and a competition is performed in an ascending order of the number of the parameters is described below. In the present modified example, it can be considered that the timing of the determination of the high-ranking condition and the calculator completion condition in the basic flow is divided into a plurality of timings. FIG. 4 is a flowchart illustrating a flow of control in the modified example of the suggestion processing according to the embodiment of the present disclosure.

In the present processing, similarly to the basic flow, after initialization (step S201) and generation of candidates (step S202) are performed, the processing is repeatedly executed in order for stage t = 1, 2, ..., and k (step S301).

The suggestion device 101 repeats the following processing for only the number of times according to the stage t (step S302). The number of times of repetition can also be set to a constant regardless of a value of t. The number of times of repetition can also be set in such a way as to be reduced as a value of t increases.

In other words, the calculator 102 executes processing similar to that in steps S204 to step S225 only on an extracted calculation candidate in which a parameter number related to a combination is t, and the calculator 102 calculates or estimates a rating value of the candidate having the parameter number t (step S303).

Then, the remover 105 removes a candidate having a low-ranking rating value among the candidates having the parameter number t from the candidate storage area 122 (step S304), and narrows down the candidate. The candidate to be removed may be limited to an estimation candidate having a low-ranking rating value, or may be both of a calculation candidate and an estimation candidate having a low-ranking rating value.

A target of deletion can also be set as, for example, candidates up to a rank in a predetermined proportion (for example, half, 1/3, 1/4, and the like) counted from a lowest rank of rating when, for example, remaining candidates having the parameter number t are sorted in an order of a rating value. The proportion may be adjusted according to the number of times of repetition for the stage t.

When repetition (step S305) ends, a predetermined number (for example, three to five) of candidates having a high-ranking rating value among the candidates having the parameter number t is selected as high-ranking candidates (step S306). In the present aspect, a round-robin competition between high-ranking candidates is subsequently performed, and thus a candidate that is even an estimation candidate can be selected as a high-ranking candidate in this stage.

Then, when repetition for each stage (step S307) ends, the round-robin competition between the high-ranking candidates is performed, and a rating value of the high-ranking candidate is calculated (step S308). By the processing, all of the high-ranking candidates become calculation candidates, and an estimation model is learned.

Next, a predetermined number (for example, ten) of the candidates having the high-ranking rating value among the high-ranking candidates is output as useful candidates (step S309), and the present processing ends.

Note that, by performing estimation and update of a rating value in step S303 on not only the estimation candidate having the parameter number t but also an estimation candidate having a parameter number (t + 1), a rating value of the candidate having the parameter number (t + 1) can be appropriately adjusted before a competition is performed in a stage (t + 1).

Also in step S304, for a target of removal, a candidate having a low-ranking rating value among the candidates having the parameter number t or (t + 1) may be removed. In this case, the candidates having the parameter number (t + 1) can be narrowed down before the competition is performed in the stage (t + 1).

Experimental Example 1

Hereinafter, based on 13 parameters of X1, X2, ..., X9, XA, XB, ..., and XD, a target in which a label y was determined nonlinearly as in

$$y = 1, \text{ if } 0.5 > X1*\sin(X2)/X3 \text{ and } X1*\sin(X2)/X3 > -0.1;$$

= 0, otherwise.

In this target, since the label y depended on X1, X2, and X3, factor parameters were X1, X2, and X3.

Thus, the following settings were applied to the embodiment described above in such a way that 300 records for determining the label y by the determination equation described above were prepared, based on a random value by using 13 parameters as the random value, representative hyperparameters were adopted into estimation models in classifications being RF, Ridge, and Lasso, and an upper limit of a parameter number was k = 4. Then, the following useful candidates were acquired as a result from a record having the above-described hidden relationship.

The following list indicates useful candidates as in "estimation model classification (factor parameter) ... rating value".

| | |
|---|---|
| RF (X1, X2, X3) | 1898.964893 |
| RF (X1, X2) | 1836.415016 |
| Ridge (X1, X3, X9) | 1712.232568 |
| Ridge (X1, X3) | 1684.863008 |
| Ridge (X1, X6, XC, XD) | 1441.144343 |
| Ridge (X1) | 1415.236034 |
| Ridge (X1, X6, X8, XD) | 1404.945883 |
| Ridge (X1, X5, X9, XB) | 1382.644057 |
| Ridge (X1, X5, X7) | 1376.231578 |
| RF (X2, X4) | 1352.316014 |
| RF (X2) | 1289.258478 |
| Ridge (X3) | 1205.748127 |

Further, a proportion of being included in a useful candidate in which a combination of parameters appears is indicated as follows.

| | |
|---|---|
| X1 | 75% |
| X2 | 33.3% |
| X3 | 33.3% |
| X1, X3 | 25% |
| X1, X2 | 16.7% |
| X1, X5 | 16.7% |
| X1, X6 | 16.7% |
| X1, X9 | 16.7% |
| X1, XD | 16.7% |
| X1, XD, X6 | 16.7% |
| X5 | 16.7% |

(continued)

| | |
|---|---|
| X6 | 16.7% |
| X9 | 16.7% |
| XD | 16.7% |
| XD, X6 | 16.7% |

In this way, a combination of parameters in useful candidates in a highest rank of rating is X1, X2, and X3 that also constitute high ranks of a parameter appearance rate. Therefore, factor parameters were properly suggested together with an appropriate classification of an estimation model, and accuracy of the present embodiment could be confirmed.

Experimental Example 2

Hereinafter, for a patient suspected of prostate cancer,

a three-dimensional feature vector acquired by performing dimensionality compression on a prostate echo image, results of the above-described 13 kinds of the blood examinations, and
whether a Gleason score determined by observing, by a doctor and the like, a pathological sample of a biopsy collected from the patient is 8 or more (whether a tumor is highly malignant)

were prepared as 87 records.

Then, the embodiment described above suggested a blood examination that needed to be combined with a prostate echo image when whether the patient had pathological malignant prostate cancer was desired to be predicted.

Similarly to Experimental Example 1, estimation models adopted representative hyperparameters and were RF, Ridge, and Lasso. With an upper limit of a parameter number as k = 5, which blood examination needed to be performed was suggested.

Results were as follows. Note that each useful candidate is indicated together with a rating value while connecting, by an underline, "RF" being a classification of an estimation model, "echo" representing use of a prostate echo image, and a name of each blood examination.

| | |
|---|---|
| RF_echo_PSA_WBC_Hb | 1635.710833 |
| RF_echo_PSA_WBC | 1578.544444 |
| RF_echo_PSA_Hb | 1575.473352 |
| RF_echo_PSA_WBC_Hb_Alb_Ca | 1532.266206 |
| RF_echo_PSA_WBC_Hb_CRP | 1520.272607 |
| RF_echo_PSA_WBC_Hb_Alb | 1516.125097 |
| RF_echo_PSA_Hb_cre | 1509.518065 |
| RF_echo_PSA_WBC_Hb_Plt | 1507.569392 |
| RF_echo_PSA_Hb_GOT | 1502.329218 |
| RF_echo_PSA_WBC_Hb_GPT _cre | 1497.454263 |

| | |
|---|---|
| PSA | 84% |
| Hb | 72% |
| PSA, Hb | 64% |
| PSA, WBC | 48% |
| PSA, WBC, Hb | 44% |

Furthermore, by calculating an AUC value 100 times by RF and calculating an average value, whether pathological malignant cancer could be predicted from these examination values was acquired as the following results.

| | |
|---|---|
| RF: echo image (PSA, WBC, Hb) | 0.828 |
| RF: echo image (PSA, Hb) | 0.818 |
| RF: echo image (PSA) | 0.795 |
| RF: echo image (Hb) | 0.687 |

(continued)

RF: echo image (WBC)                    0.622

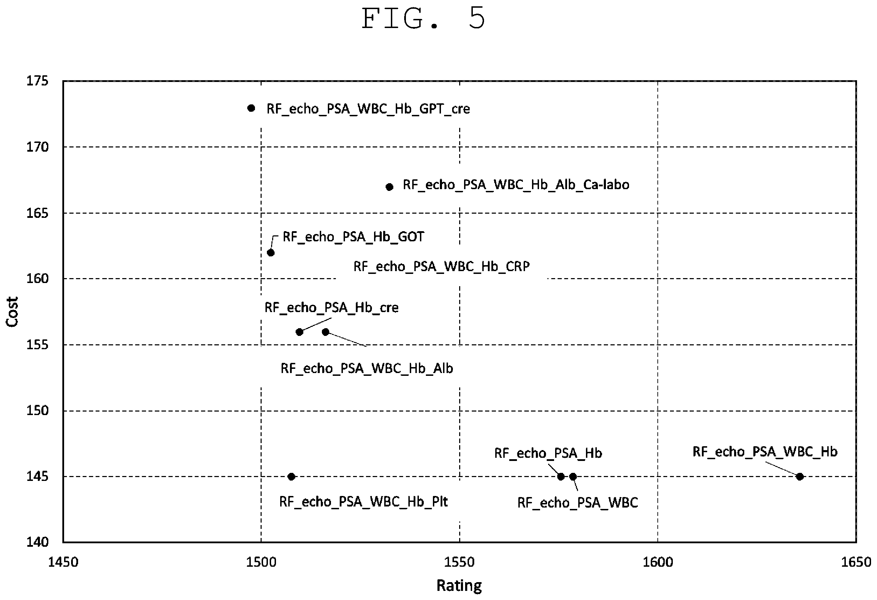

In present Experimental Example 2, the results were output to a scatter diagram. FIG. 5 is an explanatory diagram illustrating an output example of outputting a useful candidate by a scatter diagram. In the scatter diagram illustrated in FIG. 5, a vertical axis represents a cost (Cost), and a horizontal axis represents a rating value (Rating). Therefore, a cost and a rating value of a useful candidate are indicated by a dot in the scatter diagram. As a position of the dot is closer to a right lower side in the scatter diagram, cost performance of the useful candidate is more excellent.

In this way, a fact that combining WBC (white blood cell count), Hb (hemoglobin), and PSA (tumor marker) was useful for a prostate echo image, that is, these were prognosis factors, was acquired as new knowledge from present Experimental Example 2.

In Experimental Example 2, an echo photograph and (a Gleason score obtained from) a pathological photograph based on an invasive examination were used in training data. Meanwhile, a prediction was assumed to be made before an invasive operation was performed. Therefore, an echo photograph was used but a pathological photograph was unnecessary for making a prediction.

In other words, in Experimental Example 2, a prostate echo image was necessary, and any of the blood examinations was combined, but the other medical image including a pathological photograph image was not combined.

In Experimental Example 2, a combination range was more limited and a calculation amount and a calculation time were further reduced than in a case of suggesting a combination of any medical image and any of blood examinations and a case of suggesting a combination of presence or absence of an echo photograph image and any of blood examinations. In general, limiting a combination range is useful in a point of reducing a calculation amount and a calculation time required until a result of suggestion is acquired.

For example, when only a "combination including a certain fixed parameter" (a certain fixed examination is certainly performed) is set as an initial candidate, the number of candidates can be suppressed, and a calculation time and a calculation amount can be reduced.

As also clear from Experimental Examples 1 and 2, while the number of competitions is suppressed and accuracy is high, factor parameters can be suggested by adopting a rating model in the present embodiment.

Also, for an image using a feature parameter and a feature vector together with a combination of parameters such as a kind of a blood examination, for example, the number of initial candidates when only presence or absence of an "echo photograph image" is included in a combination is smaller than the number of initial candidates when presence or absence of an "echo photograph image" and presence or absence of a "pathological photograph image" are included in a combination, and the number of initial candidates is further reduced by limiting an "echo photograph image" to the presence. In this way, a calculation time and a calculation amount can be reduced by certainly using a certain kind of an image for a combination.

An examination value of a blood examination is generally known to have variations by each hospital and variations by an examination apparatus, but an influence of overlearning of an estimation model is conceivably suppressed by using a prediction value of rating estimated by deep learning.

Further, in the present embodiment, various models can be adopted as estimation models, and an appropriate hyperparameter can also be suggested.

Further, according to the present embodiment, a combination of a feature parameter and a feature vector acquired from a pathological photograph image and an echo photograph image are combined and then applied to an estimation model related to a useful candidate, and thus a highly accurate prediction can be made.

Furthermore, according to the present embodiment, data such as various medical images and various examination results that have often been handled separately can be transversely and integratedly analyzed, and a prognosis prediction more conforming to an individual patient can be achieved.

As far as the inventor can know, a technique for suggesting a prognosis factor parameter of a disease of a patient (target), based on an echo photograph image and various examination results of the patient (target), by using an artificial intelligence technology (such as machine learning and deep learning), and making a highly accurate prognosis prediction of the disease based on a prediction value (rating score) in which usefulness is verified is the first.

Output to Screen

As described above, the outputter 104 can output a useful candidate to a screen by a scatter diagram. Hereinafter, a display example onto the screen is described.

A plurality of aspects of an output of the scatter diagram from the outputter 104 is assumed such as display on an individual electronic medical chart, display on an analysis screen of an electronic medical chart system, and display on a database system instead of being limited to an electronic medical chart and an electronic medical chart system.

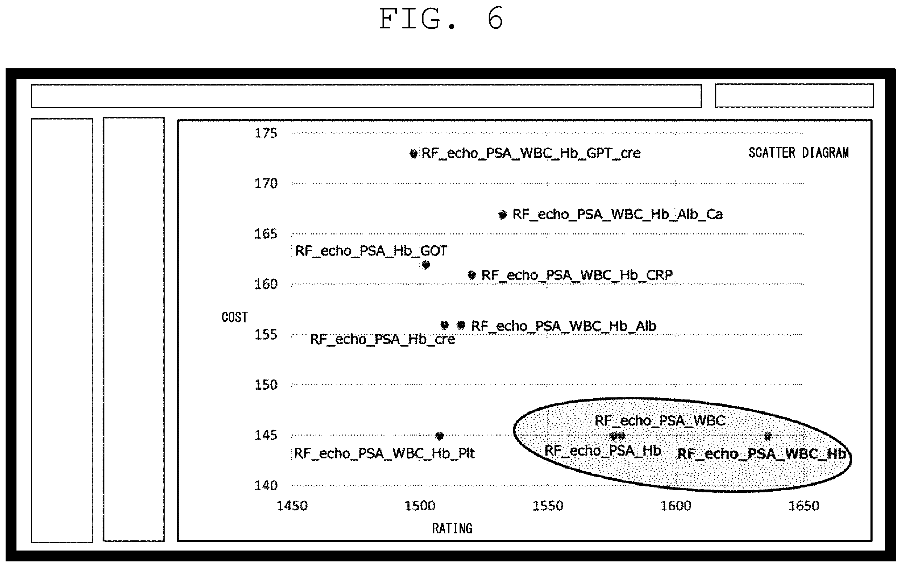

FIG. 6 is an explanatory diagram illustrating a display example of outputting the useful candidate in the scatter diagram and displaying the useful candidate on the screen. FIG. 6 illustrates an example in which electronic medical chart information and various data sets are displayed on the screen, and the scatter diagram is displayed together with the other information. An elliptical mark entered by a doctor is displayed over the scatter diagram, and a fact that the doctor has paid attention to the combination is clear. A predetermined rating score and a predetermined cost may be set in advance in the suggestion device, and the useful candidate in the set range may be automatically surrounded by an elliptical mark on the electronic medical chart screen, or the useful candidate may be displayed in an identifiable manner by a color, a mark, and the like different from the other candidate.

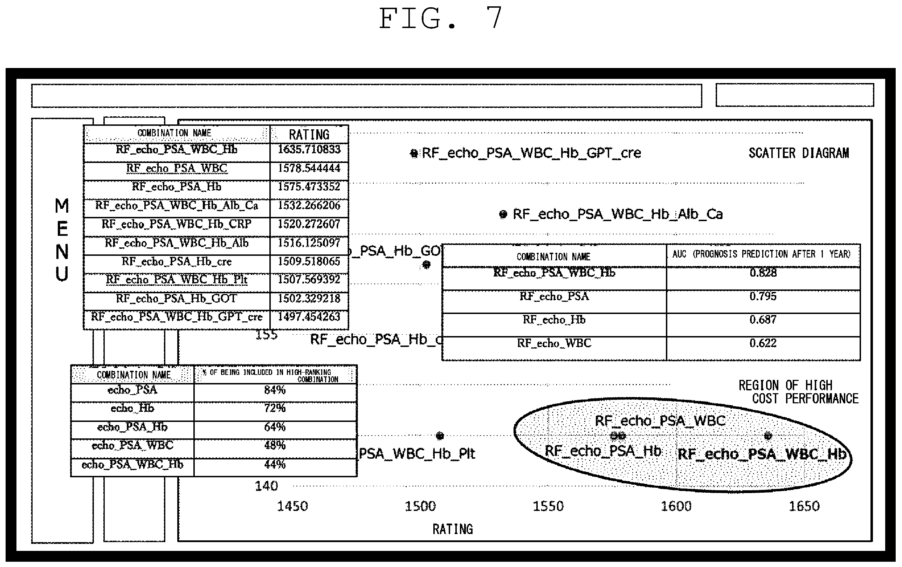

FIG. 7 is an explanatory diagram illustrating a display example of outputting the useful candidate in the scatter diagram and displaying the useful candidate on the screen. FIG. 7 is a display example for a doctor to confirm more specific information for information displayed in the scatter diagram. When the doctor performs a predetermined operation, for example, selects an item from MENU on the left of the screen, a rating score of a combination displayed in the scatter diagram or a ratio (percentage) of being included in a high-ranking combination is displayed in a sub-window, and thus the doctor can confirm specific information.

By clicking a region of high cost performance in the scatter diagram with a mouse, an AUC (prognosis prediction after 1 year) of a combination in the region of high cost performance is displayed in the sub-window, and thus prediction accuracy can be confirmed.

In addition, a medical photograph image of a combination desired to be confirmed can also be displayed in the sub-window by a predetermined operation (not illustrated).

Various new interfaces (the scatter diagrams of FIGS. 6 and 7 and an information providing function of the scatter diagrams) provided by the technique according to the present disclosure for highly accurately predicting a prognosis of a disease in which usefulness is verified are useful in a diagnosis by a doctor for appropriate medication and medical treatment having high cost performance according to a specific patient (target).

Example to which Present Embodiment is Applicable

In Experimental Example 2 described above, the example of combining an examination result of a patient having prostate cancer and a medical image constituted by an echo photograph is described, and the present embodiment can be applied to various aspects as follows.

First, in the present embodiment, for a patient who may be a target, inflammatory diseases and degenerative diseases that are non-neoplastic diseases can be applied as general diseases other than cancer. For example, autoimmune diseases, infectious diseases, wound (healing), neurodegenerative diseases, new infectious diseases that cause a pandemic of ulcerative colitis (disease that may adapt), keloid after a wound, and the like can be applied.

Furthermore, for a patient who may be a target, almost all of cancers (including all malignant tumors such as leukemia in addition to "cancers" being epithelial tumors) such as breast cancer, lung cancer, liver cancer, pancreatic cancer, bile duct cancer, esophagus cancer, stomach cancer, colon cancer, bladder cancer, kidney cancer, skin cancer, cervical cancer, uterine body cancer, ovarian cancer, testicular cancer, sarcomas, hematological tumors, brain tumors, and prostate cancer can be applied.

As an image in an aspect using various examination results and a feature parameter and a feature vector acquired by performing dimensionality compression on an image, an electron microscope image, a three-dimensional tissue image and a three-dimensional cell image using a transparent tissue, and the like can be adopted. A cytodiagnosis image, an immunohistochemical image, a fluorescent staining image, an image of a technique for detecting a distribution and an amount of DNA and mRNA by using in situ hybridization, and the like can also be adopted. In addition to various photographs before an operation, various pathological images used in a department of diagnostic pathology after an operation can also be adopted.

Furthermore, the image may also be applied to an image acquired by CT, MRI, PET, skeleton sintigraphy, a simple X ray, mammography, dermoscopy, a fundus photograph, and a corneal photograph, in addition to an echo photograph image.

Suggestion System

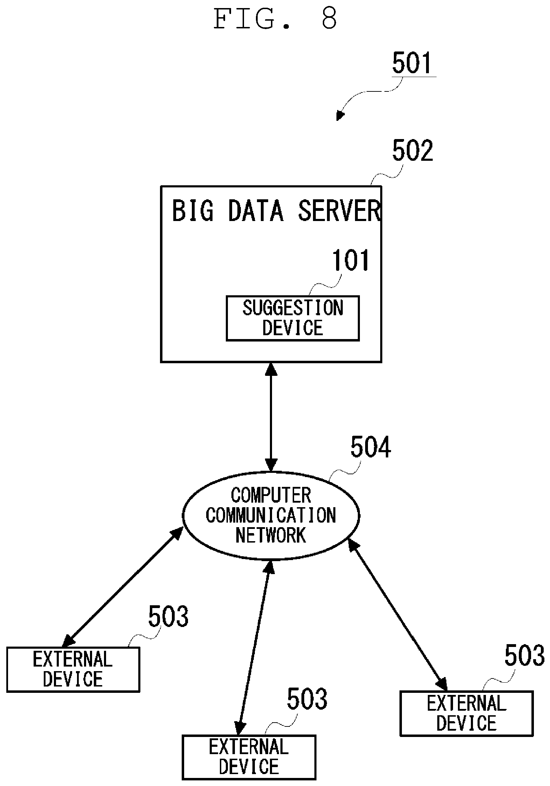

An embodiment of a suggestion system using the suggestion device described above is described below. FIG. 8 is an explanatory diagram illustrating an overview configuration of the suggestion system according to the embodiment of the present disclosure.

As illustrated in FIG. 8, in a suggestion system 501, a big data server 502 and an external device 503 are connected to each other via a computer communication network 504 such as the Internet.

The big data server 502 functions as the suggestion device 101 described above, and also executes various types of data processing and data management.

The external device 503 is achieved by a computer used in a medical treatment and examination institution such as a hospital, a research and analysis institution such as a university, and the like. Each external device 503 may be constituted by one computer, or may be constituted by a combination of an organization server that mediates between the big data server and the external device, and a terminal serving as an interface between the organization server and a doctor, a researcher, and the like.

An examination result value of a patient and a medical image of a patient are accumulated in the external device 503 used in a city hospital and a university hospital. The records recorded in the external device 503 are subjected to anonymization, then transmitted to the big data server 502, and accumulated and managed in a database in the big data server 502.

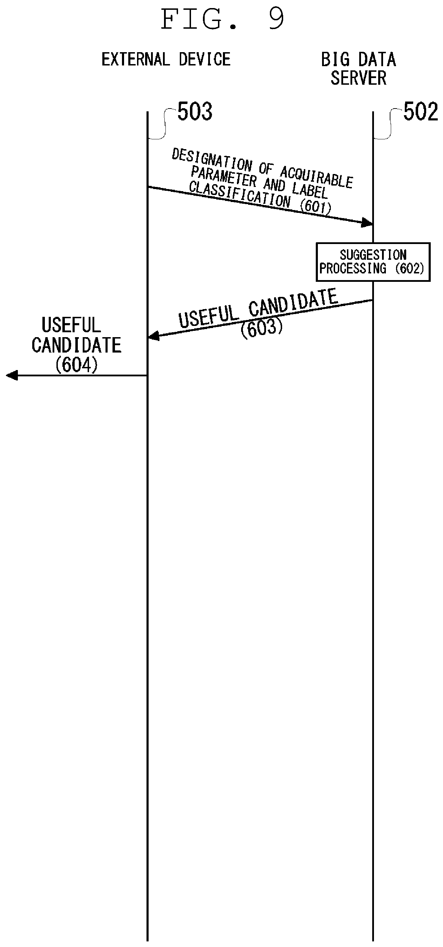

The big data server 502 functions as the suggestion device 101 described above. Herein, the suggestion device 101 achieved by the big data server 502 can function as follows. FIG. 9 is an explanatory diagram illustrating a scene of transmission and reception of information in the suggestion system according to the embodiment of the present disclosure.

In other words, designation of a "parameter that can be acquired for a target" and a "classification of a label" in the suggestion device 101 can be received from the external device 503 (601).

For example, when an examination that can be performed in a hospital and the like is limited, designation of an examination that can be performed as a "parameter that can be acquired for a target" may be made from the external device 503 to the big data server 502.

A "classification of a label" varies depending on a request purpose of a hospital and the like, that is, what kind of a purpose the present system 501 is desired to be used. For example, when a prognosis prediction of a recurrence of cancer within 1 year is desired to be made, "presence or absence of a recurrence within 1 year or a recurrence prediction value (percentage representing a probability of a recurrence)" is a label that needs to be provided to a target. Designation of a "classification of a label" related to a request purpose can also be made from the external device 503 to the big data server 502. Note that, when a prognosis prediction of a recurrence of cancer within 3 years is desired to be made as a request purpose, "presence or absence of a recurrence within 3 years or a recurrence prediction value (percentage)" is a label that needs to be provided to a target, and labels in various classifications based on the request purpose may be set.

When the big data server 502 accepts the designation described above, the big data server 502 functions as the suggestion device 101 described above, executes the suggestion processing (602), and outputs a useful candidate and the like.

Note that the big data server 502 may include a model (useful candidate) that learns from various medical images and examination results of many patients (targets) and is prepared in advance according to a typical purpose. In this case, when a purpose of a prognosis prediction is input, a model (useful candidate) that matches a purpose and is prepared in advance can be immediately output by reusing a result of the suggestion processing in the past.

Further, several typical purposes such as a prognosis prediction of a recurrence within 1 year of prostate cancer and a prognosis prediction of a recurrence within 3 years of prostate cancer may be prepared, and prognosis prediction models according to the typical purposes may be set as applications in the big data server 502. A user (such as a doctor) may access the big data server 502 from a screen of an electronic medical chart system, and set the applications usable according to a purpose.

The useful candidate is transmitted from the big data server 502 to the external device 503 (603), and performance and a rating value of the useful candidate, a parameter number, a total cost, and the like are presented (604) in the external device 503, and can be used as a reference when a doctor and the like determine future treatment and examination plans.

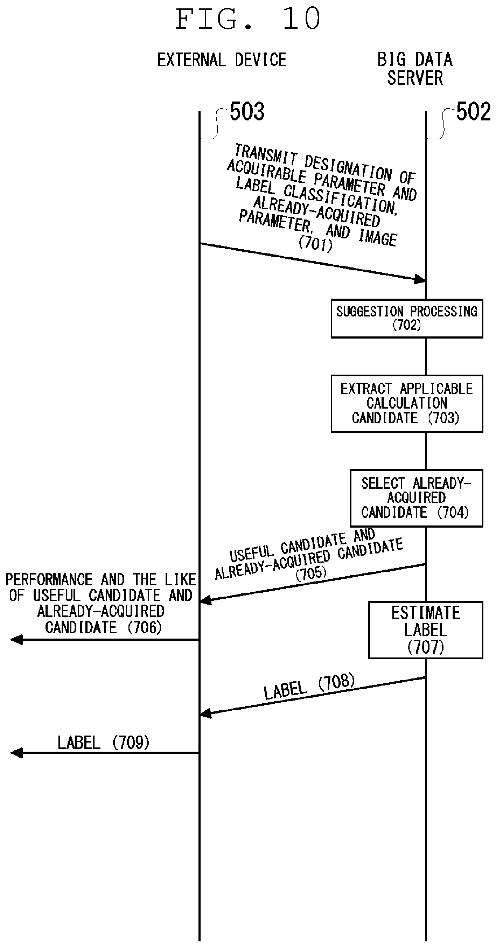

For a specific patient (target), there may be an examination value (parameter value) that has already been examined and various medical photograph images (images). At this time, when a doctor gives consideration to an electronic medical chart of the patient, and the like, processing as follows can be executed. FIG. 10 is an explanatory diagram illustrating a scene of transmission and reception of information in the suggestion system according to the embodiment of the present disclosure.

In other words, a parameter value that has already been acquired for the target, and an image are transmitted from the external device 503 to the big data server 502 together with designation of a kind of an examination that can be performed (an acquirable parameter) and a request content (a classification of a label) (701).

The big data server 502 acquires an output of a useful candidate from the suggestion processing (702).

A calculation candidate to which the transmitted image and a combination of the transmitted already-acquired parameter values can be applied, that is, a calculation candidate representing a partial combination being a part or a whole of a combination of the already-acquired parameters is extracted (703).

Note that, in an aspect in which a useful candidate being prepared in advance is output, a calculation candidate created in the advance preparation is also cached, and extraction from the cache is performed, and thus a calculation candidate representing a partial combination being a part or a whole of a combination of already-acquired parameters can be extracted in a short time.

Next, a calculation candidate having best performance or a best rating value (hereinafter referred to as an "already-acquired candidate") is selected from among the extracted calculation candidates (704). The performance or the rating value of the already-acquired candidate being acquired herein is transmitted together with information about the useful candidate to the external device 503 (705). The external device 503 presents, to a client, the performance and the rating value of estimation based on the already-acquired parameter value for the target (706).

Furthermore, subsequent to the processing described above, the big data server 502 can provide, as an input, the transmitted already-acquired parameter value and the transmitted image to an estimation model for the already-acquired candidate (707), and can also cause the estimation model to estimate a label.

The label estimated herein is transmitted to the external device 503 (708). The external device 503 presents, to the client, the transmitted label as the label estimated based on the already-acquired parameter value for the target (709). Accuracy of the label presented herein can be acquired from the performance and the rating value of the already-acquired candidate being similarly presented (706) to the client.

Furthermore, as a likelihood of the label, a probability of being classified as the label can also be used. As described above, an estimation model calculates a probability of being positive (or negative), compares the probability with a threshold value, and thus outputs a positive or negative label. Therefore, a probability of a label can be transmitted together with the label and presented to a client. "Presence or absence of a recurrence within 1 year" is used for predicting whether to come under any of two kinds of labels, and is acquired by comparing a probability output from an estimation model with a threshold value. Further, as a "recurrence prediction value (percentage representing a probability of a recurrence)", a probability output from an estimation model can be used as it is.

When a client thinks that accuracy (such as performance and a rating value) of an already-acquired candidate is sufficient, a label presented herein (and a probability of the label) can be used as a reference for a prognosis prediction of a patient.

When a client thinks that performance and the like of an already-acquired candidate is insufficient, the client refers to a useful candidate output from the big data server 502 to the external device 503, and determines an examination that needs to be additionally performed, and the like.

For making use in this decision, the external device 503 can present, to a doctor and the like, a difference in performance and a rating value between a useful candidate and an already-acquired candidate together with an examination being additionally performed in order to apply the useful candidate, and an additional cost of additional capturing of a medical image and the like. The presentation of the additional examination item, the additional medical image, and the rating score and the cost thereof may be displayed on a screen of an electronic medical chart, an electronic medical chart system, a database system, and the like as one of the information provision functions of the scatter diagram described above. Details of this is described below.

In the scatter diagram illustrated in FIG. 7 and the like,

an already-acquired candidate, and
a useful candidate having more excellent performance and rating value and having higher parameter number and cost than the already-acquired candidate may be displayed in an emphasized manner, and may both be able to be compared.

Then, a doctor and the like can take an additional cost and an improvement in accuracy into consideration, and determine which examination is to be additionally performed.

When a new parameter is acquired for a target, the processing described above may be repeated. In other words, by transmitting, again to the big data server 502, an already-acquired parameter value for the target and an image from the external device 503 to the big data server 502, a label being more highly accurately estimated can be acquired from the big data server 502.

In order to facilitate understanding, an example in which Experimental Example 2 is applied to the present system 501 is considered.

When a certain patient has undergone a prostate echo photograph and examinations of PSA and Hb but has not undergone an examination of WBC, candidates related to a partial combination are

RF_echo_PSA,
RF_echo_PSA_Hb, and
RF_echo_Hb.

Of these, RF_echo_PSA_Hb and RF_echo_Hb are useful candidates ranked in top 10, but RF_echo_PSA Hb has a higher rating value. Further, RF_echo_PSA is not a useful candidate. RF_echo_PSA_Hb has the best performance.

Therefore, an already-acquired candidate is RF_echo_PSA_Hb. The rating value of RF_echo_PSA_Hb is 1575.473352, and an AUC value is 0.818.

As a useful candidate having better rating value and performance than the already-acquired candidate, there is RF_echo_PSA_WBC_Hb (rating value: 1635.710833, AUC value: 0.828). Thus, a doctor and the like can refer to the performance and the rating value of RF_echo_PSA_Hb being the already-acquired candidate and RF_echo_PSA WBC_Hb being the useful candidate.

In this example, the number of examinations is increased by 1 and a cost of only a drug price of an additional examination of WBC is increased by performing the additional examination of WBC, but it is clear that, for a prognosis prediction label to be acquired, a rating value is increased only by 1635.710833 - 1575.473352 = 60.237481 and an AUC value is increased only by 0.828 - 0.818 = 0.010.

Thus, it is assumed that a doctor and the like take an increase in cost and an increase in likelihood of a prediction result into consideration and decide that the additional examination of WBC is to be performed. At this time, the doctor can also take the number (1 in the present example) of examinations to be additionally performed and an additional cost (drug price of the examination of WBC) into consideration.

When WBC is acquired as an additional examination value by performing the additional examination on the patient, WBC is transmitted as a "new already-acquired parameter value" together with an examination value that has already been examined in the past from the external device 503 to the big data server 502, and thus the already-acquired candidate becomes RF_echo_PSA WBC_Hb, and a label estimated by the AUC value of 0.828 for the patient, that is, a material for a more highly accurate prognosis prediction can be acquired.

Note that a candidate including an examination result and a pathological image that are impossible to acquire at a point in time of making a prognosis prediction may be excluded from useful candidates. For example, even before an operation, a candidate including a total extirpation pathological image that can be acquired only after the operation is excluded from useful candidates by determination of the big data server 502 based on a request purpose. When MRI cannot be used in a hospital of an isolated island and the like, a candidate including an MRI image is excluded from useful candidates by determination of the big data server 502 based on hospital information about a request source of a prognosis prediction.

In this way, by a function of suggesting an additional examination item candidate and an additional medical image that have a possibility of acquiring a higher prediction value (rating score) and have not been provided yet in addition to suggesting a prognosis factor parameter of a disease based on a medical image and various examination results of a patient (target) that have been provided, a further improvement in accuracy of a prognosis prediction of a disease of the patient (target) can be achieved. By presenting an additional examination item to be suggested and an additional cost due to an additional medical diagnosis, a determination material having options in consideration of a situation and the like of a patient (target) can be provided when necessity of an additional examination and image acquisition, an additional examination item, and an additional medical image are decided by a doctor.

Order of Competitions

In the example described above, in the stage t (t = 1, 2, ..., k), a candidate in which the number of parameters related to a combination is t is extracted and caused to compete (see steps S302 to S306). In other words, an order is adopted in which

a candidate in which the number of parameters is 1 is first caused to compete,
a candidate in which the number of parameters is 2 is caused to compete next,
a candidate in which the number of parameters is 3 is subsequently caused to compete,
a candidate in which the number of parameters is 4 is then caused to compete,
...
a candidate in which the number of parameters is (k - 3) is caused to compete next,
a candidate in which the number of parameters is (k - 2) is subsequently caused to compete,
a candidate in which the number of parameters is (k - 1) is then caused to compete, and
a candidate in which the number of parameters is k is lastly caused to compete. However, this order can be freely changed.

In general, a candidate in which the number of parameters related to a combination is $a_t$ can be caused to compete in the stage t by using permutations of $a_1$, $a_2$, ..., $a_k$ in which integers from 1 to k are arranged. The example described above represents a fact that $a_t = t$ is adopted as permutations. In the permutations, elements are arranged from 1 in an ascending order incremented by 1.

In addition, an order in which odd-numbered elements in permutations are arranged from 1 in an ascending order incremented by 1 and even-numbered elements in the permutations are arranged from k in a descending order decremented by 1 can be adopted. In other words, an aspect is adopted in which repetition is performed like double envelopment as in

a candidate in which the number of parameters is 1 is first caused to compete,
a candidate in which the number of parameters is k is caused to compete next,
a candidate in which the number of parameters is 2 is subsequently caused to compete,
a candidate in which the number of parameters is (k - 1) is then caused to compete,
a candidate in which the number of parameters is 3 is caused to compete next,
a candidate in which the number of parameters is (k - 2) is subsequently caused to compete,

Permutations in this case are defined as in

$$a_{(2 \times m - 1)} = m,$$

and

$$a_{(2 \times m)} = k - m + 1$$

where integer m = 1, 2, ... (note that $2 \times m - 1 \leq k$ and $2 \times m \leq k$) when k is an even number.

Further, an aspect of elements in permutations in which integers from 1 to k are randomly arranged may be adopted.

Convergence to the same optimum solution instead of a local solution is conceivably prompted by changing an order of competitions as described above depending on a use and training data.

For example, convergence to an optimum solution is further improved from 75% to 90% by sandwiching the parameter number from above and below than the aspect in which the parameter number is incremented by 1 in order.

In general, convergence to an optimum solution is expected to vary depending on a use and training data. Therefore, an optimum solution can be conceivably searched without being led into a local solution by executing processing in a plurality of different permutations.

Use of Plural Combinations of Useful Candidates and Estimation Models

In the embodiment described above, a calculation candidate having a high-ranking rating value is output as a useful candidate, but "high-ranking" herein can adopt not only one rating value but also a high-ranking group of rating values.

For example, the suggestion device 101 may select a plurality of combinations of useful candidates and estimation models of the useful candidates from ones having a high-ranking rating value.

Then, in the suggestion system 501, an already-acquired parameter value is transmitted from the external device 503 to the suggestion device 101.

In the suggestion device 101, a part or a whole of the transmitted parameter value is provided as an input to each estimation model of a high-ranking combination, and a label is output to each estimation model.

Then, the plurality of acquired labels are transmitted to the external device 503. A user of the external device 503 can acquire an impression to what extent labeling in the suggestion device 101 is reliable by whether all labels output from different estimation models coincide, and how a distribution of the labels is when the labels are different.

For example, when it is rare that a plurality of labels output to examination values that have been performed do not coincide, and a label output to an examination value this time does not coincide, an abnormal value is conceivably included in the examination value this time.

Further, when there have been many cases where a plurality of labels output to examination values that have been performed do not coincide, it can be expected that an abnormal value has been included in training data.

In general, due to a great number of combinations in a multimodal analysis, a synergistic effect of anomalies in which an abnormal prediction occasionally occurs by a combination of slight abnormal values even without occurrence of a great abnormal value (examination mistake and input mistake) in each examination may occur. In the present embodiment, risk management of an output of an estimation model can be performed by performing a pattern analysis on the output of the estimation model having a high-ranking result.

The present technique is seemingly similar to a conventional ensemble analysis in a point of using a plurality of estimation models. However, a difference is that the conventional ensemble analysis aims to improve accuracy, whereas the present technique performs risk management by analyzing diversified viewpoints of different models.

For example, in general, a value within a certain section (for example, from 0 to 1) is output as an intermediate parameter inside each estimation model, the intermediate parameter is compared with a threshold value of the estimation model, and thus a label is decided.

Thus, a pattern analysis of an output can be conceivably achieved by handling an intermediate parameter in a plurality of estimation models as a vector, and detecting an abnormal value (outlier) of the vector. Various techniques such as outlier detection based on statistics such as a generalized ESD method, outlier detection based on a distance such as a Mahalanobis' distance, and outlier detection based on density ratio estimation such as One Class SVM can be applied to detection of an outlier.

As a preliminary experiment, a plurality of estimation models having a high-ranking result based on the training data described above are selected, and detection of an outlier is performed on

(1) examination values without an abnormal value,
(2) examination values including one abnormal value, and
(3) examination values including two abnormal values, and thus

10% is detected as an outlier by mistake for (1),
40% of the examination values including the abnormal value can be detected for (2), and
all of the examination values including the abnormal value can be detected for (3).

Therefore, the present embodiment using a plurality of estimation models is conceivably useful to warn a possibility that an abnormal value is included in an examination value.

Prediction System

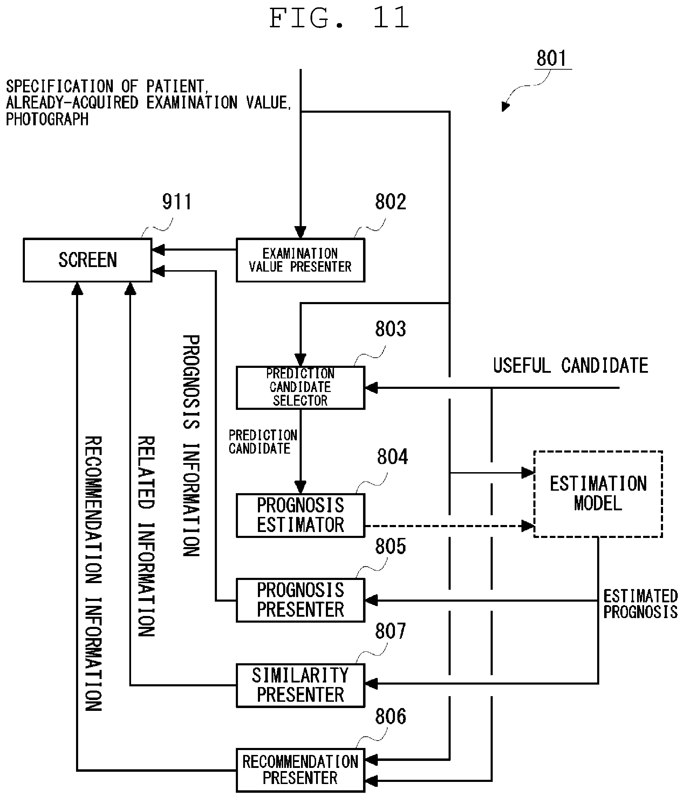

An aspect in which a part or a whole of the suggestion system 501 described above functions as a prediction system for predicting a prognosis of a patient for a medical worker who deals with the patient is described below. FIG. 11 is an explanatory diagram illustrating an overview configuration of the prediction system included in the suggestion system according to the embodiment of the present disclosure. Hereinafter, description is given with reference to FIG. 11.

As described above, a prediction system 801 takes on a part or a whole of functions of the suggestion system 501 for a medical worker who deals with a patient, and is achieved by the following roles shared among a terminal such as a personal computer used by the medical worker, a local server for locally managing an electronic medical chart and the like by a medical institution and the like in cooperation with the terminal, the big data server 502 for collecting and analyzing data from the medical institution and the like and providing various types of medical information, particularly, information about the useful candidate, the estimation model, and the like described above, and the like, is achieved by all of the following roles played by any one machine, or the like. Note that the terminal used by a medical worker, the local server that manages an electronic medical chart, and the like correspond to the external device 503 described above. Furthermore, FIG. 11 illustrates a configuration in which the prediction system 801 serves as a part of the suggestion system 501, and the suggestion device 101 itself is not included in the prediction system 801.

The prediction system 801 includes an examination value presenter 802, a prediction candidate selector 803, a prognosis estimator 804, and a prognosis presenter 805. A recommendation presenter 806 and a similarity presenter 807 may be further provided as an omittable element.

Herein, the examination value presenter 802 presents an already-acquired examination value for a patient on a screen 911 of a terminal used by a medical worker who deals with the patient.

Herein, an already-acquired examination value for a certain examination item of a certain patient means an already-acquired parameter value for a certain parameter of a certain target in the embodiment described above.

The examination value presenter 802 may further present a photograph image such as a medical photograph image and an echo photograph image that are captured for the patient.

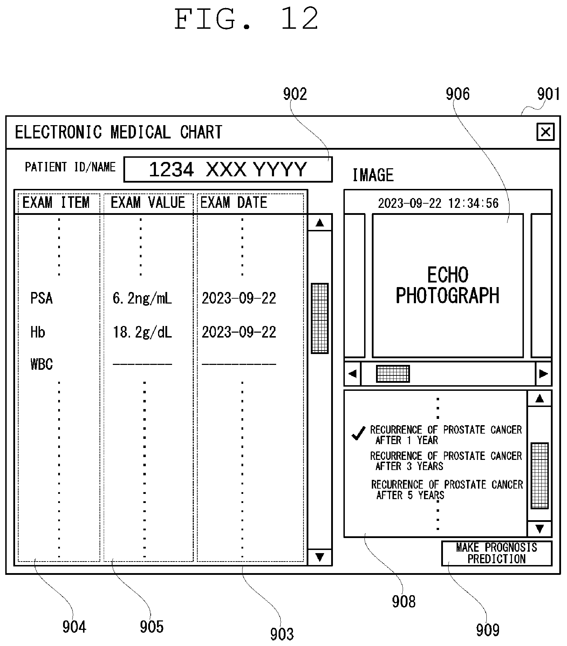

FIG. 12 is an explanatory diagram illustrating a scene of an electronic medical chart presented in the prediction system according to the embodiment of the present disclosure. Hereinafter, description is given with reference to FIG. 12.

As illustrated in FIG. 12, in an electronic medical chart 901 displayed on the screen 911 of the terminal, a name of a patient, a patient number, and the like are displayed in an identification information section 902. An examination item of the patient, and an examination value of the examination item or a fact that an examination has not been performed yet are divided into columns and displayed in an organized manner in a parameter section 904 and a parameter value section 905 of a parameter table 903. Herein, a numerical value of an already-acquired examination value is illustrated for an examination item in which an examination is performed, and a line representing that an examination has not been performed yet is illustrated for an examination item in which the examination has not been performed yet. In addition, in the example illustrated in FIG. 12, an echo photograph captured by noninvasive treatment for the patient and the like can be displayed in a photograph section 906. After invasive treatment and surgical treatment are performed on the patient, a photograph of a captured affected area, a sample photograph of a cut affected area, and the like can also be displayed in the photograph section 906.

Various types of information displayed in the electronic medical chart 901 are generally managed in a terminal used by a medical worker, a local server, and the like, but can also be stored by using a cloud service and entrusted to the big data server 502 and the like.

Hereinafter, in order to facilitate understanding, description is given to an aspect in which various blood examinations that are targeted for a patient suspected of prostate cancer and can be performed on the patient in a concerned medical institution and the like are adopted as examination items, an echo photograph image having a small physical burden on the patient is adopted as a photograph image, and a prognosis of the patient is estimated by using an estimation model related to a useful candidate and used as a material for consideration by the medical worker. However, it is needless to say that the present disclosure can be applied when various prognosis predictions are desired to be made and a determination material is desired to be acquired in an examination value of another disease and another examination item.

In the electronic medical chart 901 in the present example, a list box 908 for selecting a desired prediction and a button 909 for making the selected prediction are prepared. Note that a user interface for making a prediction is not limited to the list box 908 and the button 909 as long as the user interface has a function similar to these.

In the example in FIG. 12, various candidate items for a prognosis prediction are selectably prepared in addition to "recurrence of prostate cancer after 1 year", "recurrence of prostate cancer after 3 years", "recurrence of prostate cancer after 5 years", and the like in the list box 908.

Herein, it is assumed that the medical worker selects "recurrence of prostate cancer after 1 year" in the list box 908 and selects the button 909. Then, the prediction system 801 functions as follows.

In other words, the prediction candidate selector 803 selects, as a prediction candidate, a useful candidate in which an already-acquired examination value fulfills a combination of examination items related to the useful candidate, from a plurality of useful candidates being a plurality of useful candidates and each representing a combination of examination items.

On the other hand, the prognosis estimator 804 provides, as an input, the already-acquired examination value that fulfills the combination related to the selected prediction candidate to an estimation model that has learned the selected prediction candidate, and causes the estimation model to estimate a prognosis of the patient.

Furthermore, the prognosis presenter 805 presents the estimated prognosis on the screen 911.

Description is given below while referring to a specific example.

As described above, a plurality of useful candidates are output from the suggestion device 101, and the prediction candidate selector 803 in the prediction system 801 selects a prediction candidate from the useful candidates. Herein, one useful candidate represents a combination of parameters, and, in the present example, a combination of blood examinations applied to a patient.

When all of examination values of the blood examinations related to the combination are acquired, that is, when an already-acquired examination value fulfills a combination of examination items, a label for the patient is output by providing, as an input, the examination value that fulfills the combination of the examination items to an estimation model that has learned the effective candidate in the suggestion device 101. The label is used for estimating a prognosis of the patient.

In other words, the prediction candidate is the useful candidate with which a prediction of a prognosis can be made by using an examination value acquired for an examination item already applied to the patient. In other words, a combination related to the useful candidate corresponds to a partial combination of a combination related to the examination item already applied to the patient.

A case of making a prognosis prediction of a recurrence of prostate cancer after 1 year is considered. Herein, the recurrence is assumed to include a case where a symptom is completely gone once and then the symptom appears again, and also a case where a disease is not cured and is maintained and continued as it is.

As described above, in the training data, a label "1" (positive) and a label "0" (negative) can be respectively assigned to a case of a recurrence after 1 year and a case of no recurrence after 1 year.

In this case, when an examination value of a patient is input as a parameter value to an estimation model, a numerical value between 0 and 1 is output from a neural network and the like of the estimation model. The output value is compared with a threshold value learned together with the neural network described above and the like.

The label "0" representing no recurrence after 1 year with the output value on a side close to "0" and the label "1" representing a recurrence after 1 year with the output value on a side close to "1" are each an estimation result and a prognosis predicted for the patient.

Further, the output value compared with the threshold value can be considered to represent a degree of a recurrence after 1 year. In other words, the output value can be considered as a probability of a recurrence after 1 year or a numerical value having a positive correlation with the probability.

As described above, examples of an input to the estimation model can include, in addition to a parameter value including an examination value for an examination item, a specification (for example, may include gender, age, height, weight, presence or absence of a smoking habit, presence or absence of a drinking habit, medical history, and the like) of the patient, a photograph image of the patient itself, or a feature parameter and a feature vector acquired by performing dimensionality compression on the photograph image.

Then, for a patient being dealt by a medical worker, an examination applied to the patient represents the following.

As described above, a useful candidate represents a combination of examination items useful to predict a prognosis of a certain disease. In the example described above, a useful candidate using examination values of the blood examinations in addition to gender, age, and an echo photograph of an affected area for the patient suspected of prostate cancer is described. In other words, the example described above includes the following five as the useful candidate and performance of an estimation model of the useful candidate.

| | |
|---|---|
| RF: echo image (PSA, WBC, Hb) | 0.828 |
| RF: echo image (PSA, Hb) | 0.818 |
| RF: echo image (PSA) | 0.795 |
| RF: echo image (Hb) | 0.687 |
| RF: echo image (WBC) | 0.622 |

For example, when only a blood examination Hb is applied to the patient, one that can be set as a prediction candidate, that is, one in which only an Hb value fulfills a combination of examination items is the following one.

| | |
|---|---|
| RF: echo image (Hb) | 0.687 |

When only a blood examination PSA is applied to the patient, one that can be set as a prediction candidate, that is, one in which only a PSA value fulfills a combination of examination items is the following one.

| | |
|---|---|
| RF: echo image (PSA) | 0.795 |

Herein, in the example of the electronic medical chart 901 illustrated in FIG. 12, blood examinations Hb and PSA are applied to the patient, and a blood examination WBC is not applied. In this case, one that can be set as a prediction candidate, that is, one in which an Hb value and a PSA value fulfill a combination of examination items is the following three.

| | | |
|---|---|---|
| RF: | echo image (PSA, Hb) | 0.818 |
| RF: | echo image (PSA) | 0.795 |
| RF: | echo image (Hb) | 0.687 |

In this example, according to the embodiment, the prediction candidate selector 803

may select, as prediction candidates, all combinations fulfilled by an already-acquired examination value (in the example described above, three being RF: echo image (PSA, Hb), RF: echo image (PSA), and RF: echo image (Hb)), may select, as a prediction candidate, one having highest performance (in the example described above, one being RF: echo image (PSA, Hb)) among combinations fulfilled by an already-acquired examination value, or may select, as prediction candidates, a predetermined number or a predetermined proportion of combinations having high performance (for example, two being RF: echo image (PSA, Hb) and RF: echo image (PSA) when top two is set as a predetermined number) among combinations fulfilled by an already-acquired evaluation value.

The prognosis estimator 804 provides, for each of the selected prediction candidates, an examination value (and a specification and a photograph image of the patient) related to a combination of the prediction candidate to an estimation model related to the prediction candidate, and causes the estimation model to estimate a prognosis.

When the estimation model operates in the big data server 502, an examination value and the like of the patient are passed from the external device 503 such as a terminal and a local server to the big data server 502. Therefore, roles of the prediction candidate selector 803 and the prognosis estimator 804 can be configured to be played by the big data server 502.

On the other hand, learning of an estimation model requires using a computer having high performance such as the big data server 502, but once a learned estimation model is acquired, the learned estimation model can also operate in the external device 503 such as a terminal and a local server by acquiring configuration setting of the estimation model from the big data server 502. In this case, roles of the prediction candidate selector 803 and the prognosis estimator 804 can also be entrusted to the external device 503 such as a terminal and a local server. In addition, selection of a prediction candidate by the prediction candidate selector 803 is performed on the external device 503 side, but an estimation model itself may be prepared in the big data server 502, and the prognosis estimator 804 may control the big data server 502 on the external device 503 side and cause the estimation model to estimate a prognosis. The reason for the illustration of an estimation model surrounded by a dotted line in FIG. 11 is to indicate that the estimation model may be included in the prediction system 801 or the estimation model may be prepared outside the prediction system 801.

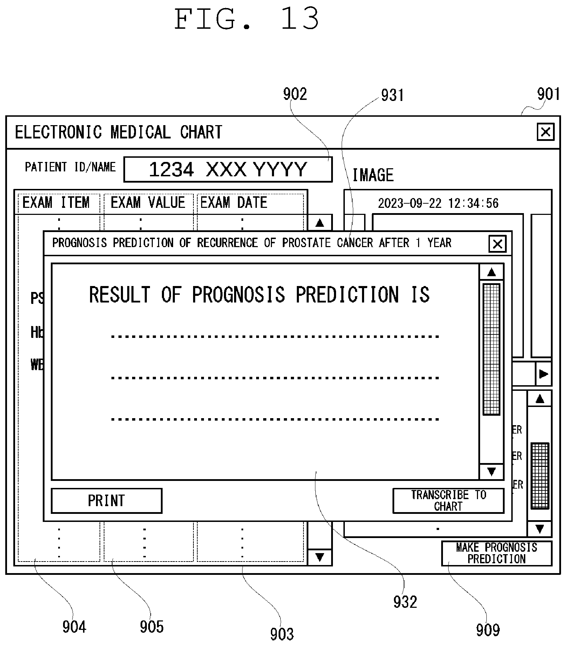

A prognosis estimated by the estimation model is presented on the screen 911. FIG. 13 is an explanatory diagram illustrating a scene where a result of a prognosis prediction is presented in the electronic medical chart presented in the prediction system according to the embodiment of the present disclosure. Hereinafter, description is given with reference to FIG. 13.

In the example illustrated in FIG. 13, a prediction result of a prognosis is displayed in a prediction result section 932 in a pop-up window 931 superimposed on the electronic medical chart 901 illustrated in FIG. 12. Note that FIG. 13 does not illustrate specific wording and the like. Hereinafter, a specific example of information displayed in the prediction result section 932 is described.

The simplest prediction result of a prognosis is an answer of only "Yes" or "No" for "recurrence of prostate cancer after 1 year" that has been selected. In addition, text including a name and performance of a used estimation model such as "According to estimation model [RF echo image (PSA, Hb)/AUC 0.818], possibility that prostate cancer of the patient recurs after 1 year is estimated low." may be described as prognosis information.

When a prognosis is estimated by a plurality of estimation models, description may be given by listing all of the estimation models and estimated prognoses.

In this case, the similarity presenter 807 may present, on the screen 911, a degree of similarity or variations of the plurality of prognoses being estimated for the plurality of selected prediction candidates, as related information being related to

reliability of the plurality of prognoses or a risk being estimated for the plurality of selected prediction candidates, and reliability or abnormality of an already-acquired examination value.

When all of the plurality of estimated prognoses coincide (or a predetermined number of the estimation models having high-ranking performance among prediction candidates coincide), that is, when variations in the plurality of prognoses are small and a degree of similarity is high, the estimation model having the highest-ranking performance and the performance of the estimation model may be indicated, and then text such as "This estimation result coincides with other two estimation models." may be notified as related information to a medical worker.

In this way, when estimated prognoses do not vary, reliability of the estimated prognoses is high and a risk is low, and it can be expected that reliability of an already-acquired examination value for the patient is also high and an abnormal value is not included.

On the other hand, when a plurality of estimated prognoses greatly vary and a degree of similarity is low, text such as "Since this estimation result does not coincide with other two estimation models, re-examination or additional examination is recommended." may be notified as related information to a medical worker. At this time, the plurality of estimated prognoses and names and performance of estimation models are also desirably presented.

Suggestion for New Examination Item

In the example described above, the five useful candidates for making a prognosis prediction of prostate cancer are acquired as described above, and the already-acquired examination values are the two being the Hb value and the PSA value. Thus, a useful candidate having highest performance among the useful candidates related to the combination fulfilled by the two examination values is "RF: echo image (PSA, Hb) ... AUC 0.818".

However, there is a useful candidate better in terms of performance than "RF: echo image (PSA, Hb) ... AUC 0.818" among the five useful candidates. In other words, the useful candidate is "RF: echo image (PSA, WBC, Hb) ... AUC 0.828".

Then, in the useful candidate of "RF: echo image (PSA, WBC, Hb) ... AUC 0.828", an examination item not fulfilled by the already-acquired examination values is WBC.

Thus, in the present aspect, the prediction candidate selector 803 may select, as a recommendation candidate from a plurality of useful candidates, a useful candidate in which performance related to a useful candidate is better in terms of performance related to the selected prediction candidate, and the recommendation presenter 806 may present, on the screen 911,

an examination item not allotted by an already-acquired examination value among combinations related to the selected recommendation candidate, and contrast information that contrasts performance related to the selected recommendation candidate with performance related to the selected prediction candidate.

For example, in the example described above, by adding, as information displayed in the prediction result section 932,

information about an examination item not allotted by an already-acquired examination value among the combinations related to the selected recommendation candidate such as "WBC is suggested as additional examination", and contrast information such as "Performance of prognosis prediction is increased by 0.010 from 0.818 to 0.828 by performing the additional examination. Price cost of the examination is XXXX.", advice on an additional examination can be given to a medical worker.

Suggestion for Whether Operation Needs to be Performed

An aspect in which advice on whether an invasive or surgical operation such as surgery needs to be performed on a concerned patient is given is described below.

In the example of prostate cancer described above, when a medical worker needs to consider whether to perform a surgical or invasive operation on a patient suspected of prostate cancer, the medical worker can receive advice, based on an estimation model according to the present disclosure.

The present aspect is configured in such a way that the estimation model estimates two prognoses of a prognosis when a surgical or invasive operation is performed on a patient and a prognosis when the operation is not performed on the patient, and

the prognosis presenter 805 presents, on the screen 911, at least any one of

the two estimated prognoses, and
whether there is a difference between the two estimated prognoses.

Thus, in the suggestion device 101, the suggestion system 501, and the prediction system 801 described above, whether the patient has undergone the predetermined operation (particularly, invasive or surgical treatment) is added as an input of an estimation model to the aspect in the example described above.

Then, in a learning stage of the estimation model, for a training example in which the operation is performed, training data are prepared in which

a parameter value of the patient related to the training example (may include a specification such as gender of the patient, an echo image of the patient, and the like), and
a parameter indicating that the patient has undergone the operation are used as input data, and
a label for classifying a prognosis of a recurrence after 1 year is used as output data (correct data). For a training example in which the operation is not performed, training data are set in which
a parameter value similar to that described above of the patient related to the training example, and
a parameter indicating that the patient has not undergone the operation are used as input data, and
a label for classifying a prognosis of a recurrence after 1 year is used as output data (correct data).

Then, similarly to the example described above, a useful candidate related to the prediction is selected in advance by performing learning of the estimation model and performing a competition between candidates related to the estimation model.

In a prediction stage in which the medical worker deals with the patient suspected of prostate cancer, the estimation model is caused to

estimate a prognosis (prognosis with an operation) by inputting a parameter value of the patient together with a fact that the patient undergoes the operation, and
also estimate a prognosis (prognosis without an operation) by inputting a parameter value of the patient together with a fact that the patient does not undergo the operation.

Then, when the prognosis with the operation and the prognosis without the operation coincide, the medical worker can acquire a material for determining that the operation does not need to be performed.

When the prognosis with the operation and the prognosis without the operation do not coincide, and the prognosis with the operation is a better prognosis, the medical worker can acquire a material for determining that it is better to perform the operation.

When the prognosis with the operation and the prognosis without the operation do not coincide, and the prognosis without the operation is a better prognosis, the medical worker can acquire a material for determining that the operation should not be performed.

Therefore, based on whether the two estimated prognoses of the prognosis with the operation and the prognosis without the operation coincide, the prognosis presenter 805 can give advice as information displayed in the prediction result section 932 of the screen 911 to the medical worker by text such as

"Prognoses after 1 year are same regardless of presence or absence of invasive or surgical treatment, and necessity to perform the treatment is thus conceivably low.",
"Prognosis after 1 year is better when invasive or surgical treatment is performed than when not being performed, and it is thus conceivably better to perform the treatment.", and
"Prognosis after 1 year is better when invasive or surgical treatment is not performed than when being performed, and it is thus conceivable that the treatment should not be performed.", and the like.

Note that, the advice described above may be displayed in a manner other than the pop-up window 931 instead of being presented by the pop-up window 931, and may be cited and included in a doctor finding section in the electronic medical chart 901 and be able to be appropriately referred by a doctor.

Prediction Processing

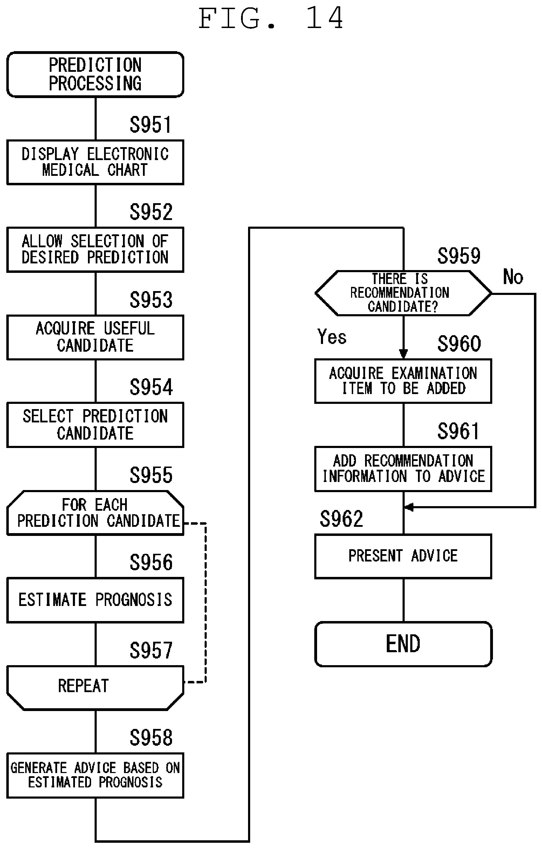

Prediction processing executed in the prediction system 801 described above is described below. FIG. 14 is a flowchart illustrating a flow of control of the prediction processing executed in the prediction system according to the embodiment of the present disclosure. Hereinafter, description is given with reference to FIG. 14.

When the present processing starts, the prediction system 801 first displays, on the screen 911, the electronic medical chart 901 including an already-acquired examination value for a patient and the like, and provides the electronic medical chart 901 to a medical worker (step S951).

Then, the prediction system 801 accepts selection of a desired prediction from the medical worker (step S952).

Next, the prediction system 801 acquires a plurality of useful candidates related to the selected prediction from the suggestion device 101 or a set of useful candidates prepared in advance in the prediction system 801 (step S953).

Furthermore, the prediction system 801 selects a prediction candidate from the acquired useful candidates, based on the already-acquired examination value for the patient (step S954).

Then, the prediction system 801 repeats processing of providing, for each of the selected prediction candidates (step S955), the already-acquired examination value of the patient as an input to an estimation model related to the prediction candidate, and causing the estimation model to estimate a prognosis (step S956) (step S957). Note that, in step S956, each of a prognosis with an operation and a prognosis without the operation may be estimated.

Next, the prediction system 801 generates advice to be provided to the medical worker, based on the estimated prognosis (step S958).

As described above, the advice generated herein can choose and include, according to a use, information about

an estimation result by the estimation model having highest-ranking performance,
an estimation result of each of the plurality of estimation models,
related information about variations and a degree of similarity of the plurality of estimation models, and reliability of a prediction and an examination value, a risk, and presence or absence of an abnormal value,
whether a prognosis is to be changed by whether an operation is performed,
and the like.

Furthermore, the prediction system 801 checks whether there is a useful candidate better in terms of performance than the prediction candidate having highest-ranking performance, that is, whether there is a recommendation candidate (step S959). When there is no recommendation candidate (step S959; No), the processing proceeds to step S962.

On the other hand, when there is the recommendation candidate (step S959; Yes), an examination item that needs to be added is acquired by excluding an examination item of the prediction candidate having the highest-ranking performance from an examination item of the recommendation candidate (step S960). Then, recommendation information acquired by choosing, for a use, information about

an examination item that needs to be added,
how much performance of the estimation model is improved by adding the examination item,
a cost for performing the examination item,
and the like is added to the advice described above (step S961).

Lastly, the prediction system 801 presents the advice on the screen 911 (step S962), and the present processing

# EP 4 597 514 A1

ends.

(Conclusion)

As described above, in order to solve the problems described above, the present disclosure is an unprecedented new technique acquired by diligently exerting originality and ingenuity by the inventor and conceived by using an artificial intelligence technology. In an aspect using the new technique for a prognosis prediction of a disease such as cancer and the like, a great effect capable of providing knowledge for appropriate medication and medical treatment having high cost performance can be acquired.

As described above, a suggestion device according to the present embodiment is a suggestion device that suggests, based on a plurality of records, any one or more candidates of a plurality of candidates each representing a combination of parameters to be acquired for a target, as a useful candidate representing a combination of factor parameters contributing to a decision of a label provided to the target, each record of the plurality of records including a plurality of parameter values each acquired by acquiring a plurality of parameters for a target associated with the each record, and a label provided to the associated target, and the suggestion device is configured to include:

a calculator that

learns, by referring to the plurality of records, an estimation model for each calculation candidate of a calculation candidate being a part of the plurality of candidates,
calculates, by the learned estimation model, performance that estimates a label provided to a target from a parameter value in which a parameter related to a combination represented by the each calculation candidate is acquired for the target, and
calculates a rating value associated with the each calculation candidate, based on the calculated performance;

an estimator that

learns a rating model by referring to a rating value calculated for the calculation candidate, and
estimates a rating value associated with each estimation candidate of an estimation candidate other than the calculation candidate among the plurality of candidates, based on the learned rating model; and

an outputter that outputs, as the useful candidate, a calculation candidate associated with a rating value being high-ranking among the calculated or estimated rating values.

The suggestion device according to the present embodiment can be configured in such a way that

each candidate of the plurality of candidates further represents a classification and a specification of an estimation model,
the calculator learns, for the each calculation candidate, an estimation model related to a classification and a specification represented by the each calculation candidate, and
the outputter further outputs an estimation model learned for the useful candidate.

The suggestion device according to the present embodiment can be configured in such a way that, after processing of setting, as the calculation candidate, an estimation candidate associated with a rating value being high-ranking among the calculated or estimated rating values, and then performing calculation by the calculator and estimation by the estimator is repeated, an output by the outputter is performed.

The suggestion device according to the present embodiment can be configured to further include

a remover that removes at least an estimation candidate having the associated rating value being low-ranking from the plurality of candidates,
wherein removal by the remover is further performed in the repeated processing.

The suggestion device according to the present embodiment can be configured in such a way that

training data for learning the estimation model are generated from a training record randomly selected from the plurality of records,
test data for calculating performance of the learned estimation model are generated from a test record being a remainder acquired by removing the training record from the plurality of records, and

the training data and the test data set,

as input data, a parameter value of a parameter related to a combination represented by the each calculation candidate among parameter values in the training record and the test record, and,

as output data, a label in the training record and the test record.

The suggestion device according to the present embodiment can be configured in such a way that

the each record further includes a feature parameter or a feature vector acquired by performing dimensionality compression on an image in which a target associated with the each record is captured, and

the feature parameter or the feature vector is included in the input data for the estimation model.

The suggestion device according to the present embodiment can be configured in such a way that, based on a rating system, the calculator repeats competition processing of

obtaining performance from two or more candidates randomly extracted from the plurality of candidates, by setting the training record and the test record to be common,

determining a competition result of causing the two or more candidates to compete, based on the obtained performance, and

updating each of rating values of the plurality of candidates, based on the determined competition result.

The suggestion device according to the present embodiment can be configured in such a way that the calculator preferentially extracts, from among the plurality of candidates, a candidate that has not learned an estimation model for the candidate or a candidate having a small number of times of learning, and thus sets the extracted candidate as the calculation candidate.

The suggestion device according to the present embodiment can be configured in such a way that the rating system is any of Elo Rating, Glicko Rating, Glicko 2 Rating, and TrueSkil (trademark).

The suggestion device according to the present embodiment can be configured in such a way that

the combination is expressed by an n-dimensional binary vector with respect to the number n of the plurality of parameters, and,

when a binary vector expressing a combination represented by the estimation candidate is input, the rating model outputs a rating value for the estimation candidate.

The suggestion device according to the present embodiment, in the suggestion device can be configured in such a way that,

for a stage $t = 1, 2, ...$, and k in order, by using permutations $a_1, a_2, ...$, and $a_k$ in which the number of parameters related to a combination represented by the useful candidate is k or less and integers from 1 to k are arranged,

a candidate in which the number of parameters related to a combination is t is extracted as the two or more candidates from the plurality of candidates, and is caused to compete in the competition processing, and,

after the competition processing is repeated, the plurality of candidates are narrowed down by the estimator and the remover.

The suggestion device according to the present embodiment can be configured in such a way that, for each candidate of the plurality of candidates, a rating value estimated in a stage t is an initial value of a rating value in a stage (t + 1).

The suggestion device according to the present embodiment can be configured in such a way that,

in the stage t, the remover removes a candidate in which the number of parameters related to a combination is t or (t + 1) and in which the estimated rating value is low-ranking,

for the stage $t = 1, 2, ...$, and k, the outputter extracts a candidate in which the number of parameters related to a combination is t and in which the updated or the estimated rating value is high, and

the calculator causes the extracted candidate to compete in a round-robin competition.

The suggestion device according to the present embodiment can be configured in such a way that elements of the permutations are arranged from 1 in an ascending order incremented by 1.

The suggestion device according to the present embodiment can be configured in such a way that odd-numbered elements in the permutations are arranged from 1 in an ascending order incremented by 1, and even-numbered elements in the permutations are arranged from k in a descending order decremented by 1.

The suggestion device according to the present embodiment can be configured in such a way that, in elements of the permutations, integers from 1 to k are randomly arranged.

The suggestion device according to the present embodiment can be configured in such a way that

the outputter outputs the useful candidate by drawing the useful candidate in a scatter diagram,
a first axis in the scatter diagram indicates the number of parameters related to a combination represented by the useful candidate, or a total cost for acquiring a parameter related to the combination, and
a second axis in the scatter diagram indicates performance or a rating value calculated for the useful candidate.

The suggestion device according to the present embodiment can be configured in such a way that

the target is a patient,
the plurality of parameters is a plurality of examination items to be applied to the patient,
the plurality of parameter values is examination result values each acquired by applying the plurality of examination items to the patient,
the label identifies a prognosis of the patient, and
the factor parameter is a prognosis factor contributing to a prediction of a prognosis of the patient.

The suggestion device according to the present embodiment can be configured in such a way that

the target is a cancer patient,
the plurality of parameters is a plurality of examination items to be applied to the cancer patient, and includes determination of a score or classification, based on a plurality of blood examinations to be applied to the cancer patient, and a medical photograph image,
the plurality of parameter values is examination result values each acquired by applying the plurality of examination items to the cancer patient,
the factor parameter is a prognosis factor contributing to a prediction of a prognosis of the cancer patient,
the image is a medical photograph image captured for the cancer patient, and
the label represents presence or absence of a recurrence in the cancer patient.

The suggestion device according to the present embodiment can be configured in such a way that

the target is a patient suspected of cancer,
the plurality of parameters is a plurality of blood examinations to be applied to the patient,
the plurality of parameter values is examination result values each acquired by applying the plurality of examination items to the patient,
the factor parameter is a factor contributing to a prediction of whether the patient has a highly malignant tumor,
the image is an echo photograph image captured for the patient, and
a label in the plurality of records is a determination result of a score or classification based on a medical sample.

A suggestion system according to the present embodiment is configured to include:

an external device; and
the suggestion device described above, wherein
the external device makes designation of the parameter to be acquired to the suggestion device,
the suggestion device outputs the useful candidate to the external device, and
the external device

accepts a combination of already-acquired parameters for a target, and
presents, for the output useful candidate, a parameter (hereinafter referred to as an "unacquired parameter") not being the accepted already-acquired parameter among parameters related to a combination represented by the useful candidate.

The suggestion system according to the present embodiment can be configured in such a way that the external device further presents, for the output useful candidate, at least any one of

the number of the unacquired parameters,
an additional cost for acquiring the unacquired parameter, and
performance or a rating value calculated for the useful candidate.

The suggestion system according to the present embodiment can be configured in such a way that

the external device further makes designation of a combination of the accepted already-acquired parameters to the suggestion device,
the suggestion device further outputs, to the external device, a candidate (hereinafter referred to as an "already-acquired candidate") having best performance or a best rating value being calculated for a calculation candidate representing a partial combination included in the designated combination, and performance or a rating value of the already-acquired candidate, and
the external device further presents the output performance or the output rating value for the already-acquired candidate.

The suggestion system according to the present embodiment can be configured in such a way that

the suggestion device,

when the already-acquired candidate is not the calculation candidate, learns an estimation model for the already-acquired candidate, and calculates performance, and
further outputs the performance calculated for the already-acquired candidate to the external device, and

the external device further presents the output performance for the already-acquired candidate.

The suggestion system according to the present embodiment can be configured in such a way that the external device presents performance or a rating value calculated for the useful candidate by a difference from performance or a rating value output for the already-acquired candidate.

The suggestion system according to the present embodiment can be configured in such a way that,

when designation of the parameter to be acquired is made, or after the unacquired parameter is presented and a new parameter for the target is acquired, the external device transmits a parameter value of an already-acquired parameter for the target to the suggestion device,
the suggestion device

provides, as input data, a part or a whole of the transmitted parameter value to an estimation model for the useful candidate, and estimates a label to be provided to the target, and
transmits the estimated label to the external device, and

the external device presents the transmitted label.

The suggestion system according to the present embodiment can be configured in such a way that

the suggestion device

outputs a plurality of combinations of the useful candidate and an estimation model learned for the useful candidate,
estimates a plurality of labels to be provided to the target by providing, as input data, a part or a whole of the transmitted parameter value to each estimation model of the plurality of combinations, and
transmits the plurality of estimated labels to the external device, and

the external device presents the plurality of transmitted labels.

The suggestion system according to the present embodiment can be configured in such a way that

the external device is a terminal for a medical worker who deals with a patient,
the parameter is an examination item to be applied to the patient,
the already-acquired parameter is an examination item that has been applied to the patient,

the unacquired parameter is an examination item that has not been applied yet to the patient,
the parameter value is an examination value to be acquired by applying the examination item to the patient,
a prognosis of the patient is classified by the label,
the terminal presents an already-acquired examination value for the patient on a screen,
the suggestion device or the terminal selects, as a prediction candidate from the plurality of useful candidates, a useful candidate in which the already-acquired examination value fulfills a combination of examination items related to a useful candidate,
the suggestion device provides, as an input, the already-acquired examination value that fulfills a combination related to the selected prediction candidate to an estimation model learned for the selected prediction candidate, and causes the estimation model to estimate a prognosis of the patient, and
the terminal presents the estimated prognosis on the screen.

The suggestion system according to the present embodiment is a prediction system for a medical worker who deals with a patient, and the suggestion system is configured to include:

an examination value presenter that presents an already-acquired examination value for the patient on a screen;
a prediction candidate selector that selects, as a prediction candidate, a useful candidate in which the already-acquired examination value fulfills a combination of examination items related to a useful candidate, from a plurality of useful candidates being a plurality of useful candidates and each representing a combination of examination items;
a prognosis estimator that provides, as an input, the already-acquired examination value that fulfills a combination related to the selected prediction candidate to an estimation model learned for the selected prediction candidate, and causes the estimation model to estimate a prognosis of the patient; and
a prognosis presenter that presents the estimated prognosis on the screen.

The suggestion system (prediction system) according to the present embodiment can be configured in such a way that

each useful candidate of the plurality of useful candidates further represents performance of the estimation model learned for the each useful candidate,
the prediction candidate selector selects, as a recommendation candidate from the plurality of useful candidates, a useful candidate in which performance related to a useful candidate is better in terms of performance related to the selected prediction candidate, and
the suggestion system further includes a recommendation presenter that presents, on the screen,

an examination item not allotted by the already-acquired examination value among combinations related to the selected recommendation candidate, and
contrast information that contrasts performance related to the selected recommendation candidate with performance related to the selected prediction candidate.

The suggestion system (prediction system) according to the present embodiment can be configured in such a way that

the selected prediction candidate is plural, and
the suggestion system further includes a similarity presenter that presents, on the screen, a degree of similarity or variations of a plurality of prognoses being estimated for the plurality of selected prediction candidates as related information being related to
reliability of a plurality of prognoses or a risk being estimated for the plurality of selected prediction candidates, and
reliability or abnormality of the already-acquired examination value.

The suggestion system (prediction system) according to the present embodiment can be configured in such a way that

the examination value presenter further presents a photograph image captured for the patient, and
a feature parameter or a feature vector acquired by performing dimensionality compression on the photograph image is further provided as the input to the estimation model.

The suggestion system (prediction system) according to the present embodiment can be configured in such a way that

the patient is a patient suspected of cancer,
the plurality of examination items is a plurality of blood examinations to be applied to the patient, and
the photograph image is a medical photograph image or an echo photograph image.

The suggestion system (prediction system) according to the present embodiment can be configured in such a way that

the estimation model estimates two prognoses of a prognosis when a surgical or invasive operation is performed on the patient and a prognosis when the operation is not performed on the patient, and
the prognosis presenter presents at least any one of

the two estimated prognoses, and
whether there is a difference between the two estimated prognoses.

A suggestion method according to the present embodiment is a suggestion method in which a suggestion device suggests, based on a plurality of records, any one or more candidates of a plurality of candidates each representing a combination of parameters to be acquired for a target, as a useful candidate representing a combination of factor parameters contributing to a decision of a label provided to the target, each record of the plurality of records including a plurality of parameter values each acquired by acquiring a plurality of parameters for a target associated with the each record, and a label provided to the associated target, and the suggestion method is configured to include:

a calculation step of

learning, by referring to the plurality of records, an estimation model for each calculation candidate of a calculation candidate being a part of the plurality of candidates,
calculating, by the learned estimation model, performance that estimates a label provided to a target from a parameter value in which a parameter related to a combination represented by the each calculation candidate is acquired for the target, and
calculating a rating value associated with the each calculation candidate, based on the calculated performance;

an estimation step of

learning a rating model by referring to a rating value calculated for the calculation candidate, and
estimating a rating value associated with each estimation candidate of an estimation candidate other than the calculation candidate among the plurality of candidates, based on the learned rating model; and

an output step of outputting, as the useful candidate, a calculation candidate associated with a rating value being high-ranking among the calculated or estimated rating values.

A program according to the present embodiment is a program causing a computer to suggest, based on a plurality of records, any one or more candidates of a plurality of candidates each representing a combination of parameters to be acquired for a target, as a useful candidate representing a combination of factor parameters contributing to a decision of a label provided to the target, each record of the plurality of records including a plurality of parameter values each acquired by acquiring a plurality of parameters for a target associated with the each record, and a label provided to the associated target, and the program can be configured to cause the computer to function as:

a calculator that

learns, by referring to the plurality of records, an estimation model for each calculation candidate of a calculation candidate being a part of the plurality of candidates,
calculates, by the learned estimation model, performance that estimates a label provided to a target from a parameter value in which a parameter related to a combination represented by the each calculation candidate is acquired for the target, and
calculates a rating value associated with the each calculation candidate, based on the calculated performance;

an estimator that

learns a rating model by referring to a rating value calculated for the calculation candidate, and

estimates a rating value associated with each estimation candidate of an estimation candidate other than the calculation candidate among the plurality of candidates, based on the learned rating model; and

an outputter that outputs, as the useful candidate, a calculation candidate associated with a rating value being high-ranking among the calculated or estimated rating values.

The program according to the present embodiment can be distributed and sold by being recorded in a non-transitory computer-readable information recording medium. Further, the program can be distributed and sold via a temporary transmission medium such as a computer communication network.

The foregoing describes some example embodiments for explanatory purposes. Although the foregoing discussion has presented specific embodiments, persons skilled in the art will recognize that changes may be made in form and detail without departing from the broader spirit and scope of the invention. Accordingly, the specification and drawings are to be regarded in an illustrative rather than a restrictive sense. This detailed description, therefore, is not to be taken in a limiting sense, and the scope of the invention is defined only by the included claims, along with the full range of equivalents to which such claims are entitled.

This application claims the benefit of Japanese Patent Application No. 2022-155487, filed on September 28, 2022, and Japanese Patent Application No. 2023-86586, filed on May 26, 2023, of which the entirety of the disclosures is incorporated by reference herein.

Industrial Applicability

The present disclosure can provide a suggestion device, a suggestion method, a suggestion system, a program, and an information recording medium for suggesting a factor parameter for estimating a label of a target among a plurality of parameters that can be acquired for the target.

Reference Signs List

| | |
|---|---|
| 101 | Suggestion device |
| 102 | Calculator |
| 103 | Estimator |
| 104 | Outputter |
| 105 | Remover |
| 121 | Database |
| 122 | Candidate storage area |
| 501 | Suggestion system |
| 502 | Big data server |
| 503 | External device |
| 504 | Computer communication network |
| 801 | Prediction system |
| 802 | Examination value presenter |
| 803 | Prediction candidate selector |
| 804 | Prognosis estimator |
| 805 | Prognosis presenter |
| 806 | Recommendation presenter |
| 807 | Similarity presenter |
| 901 | Electronic medical chart |
| 902 | Identification information section |
| 903 | Parameter table |
| 904 | Parameter section |
| 905 | Parameter value section |
| 906 | Photograph section |
| 908 | List box |
| 909 | Button |
| 911 | Screen |
| 931 | Pop-up window |
| 932 | Prediction result section |

**Claims**

1. A suggestion device that suggests, based on a plurality of records, any one or more candidates of a plurality of candidates each representing a combination of parameters to be acquired for a target, as a useful candidate representing a combination of factor parameters contributing to a decision of a label provided to the target, each record of the plurality of records including a plurality of parameter values each acquired by acquiring a plurality of parameters for a target associated with the each record, and a label provided to the associated target, the suggestion device comprising:

   a calculator that

      learns, by referring to the plurality of records, an estimation model for each calculation candidate of a calculation candidate being a part of the plurality of candidates,
      calculates, by the learned estimation model, performance that estimates a label provided to a target from a parameter value in which a parameter related to a combination represented by the each calculation candidate is acquired for the target, and
      calculates a rating value associated with the each calculation candidate, based on the calculated performance;

   an estimator that

      learns a rating model by referring to a rating value calculated for the calculation candidate, and
      estimates a rating value associated with each estimation candidate of an estimation candidate other than the calculation candidate among the plurality of candidates, based on the learned rating model; and

   an outputter that outputs, as the useful candidate, a calculation candidate associated with a rating value being high-ranking among the calculated or estimated rating values.

2. The suggestion device according to claim 1, wherein

   each candidate of the plurality of candidates further represents a classification and a specification of an estimation model,
   the calculator learns, for the each calculation candidate, an estimation model related to a classification and a specification represented by the each calculation candidate, and
   the outputter further outputs an estimation model learned for the useful candidate.

3. The suggestion device according to claim 1, wherein, after processing of setting, as the calculation candidate, an estimation candidate associated with a rating value being high-ranking among the calculated or estimated rating values, and then performing calculation by the calculator and estimation by the estimator is repeated, an output by the outputter is performed.

4. The suggestion device according to claim 3, further comprising a remover that removes at least an estimation candidate having the associated rating value being low-ranking from the plurality of candidates,
   wherein removal by the remover is further performed in the repeated processing.

5. The suggestion device according to claim 4, wherein

   training data for learning the estimation model are generated from a training record randomly selected from the plurality of records,
   test data for calculating performance of the learned estimation model are generated from a test record being a remainder acquired by removing the training record from the plurality of records, and
   the training data and the test data set,

      as input data, a parameter value of a parameter related to a combination represented by the each calculation candidate among parameter values in the training record and the test record, and,
      as output data, a label in the training record and the test record.

6. The suggestion device according to claim 5, wherein

the each record further includes a feature parameter or a feature vector acquired by performing dimensionality compression on an image in which a target associated with the each record is captured, and
the feature parameter or the feature vector is included in the input data for the estimation model.

7. The suggestion device according to claim 5, wherein,
based on a rating system, the calculator repeats competition processing of

obtaining performance from two or more candidates randomly extracted from the plurality of candidates, by setting the training record and the test record to be common,
determining a competition result of causing the two or more candidates to compete, based on the obtained performance, and
updating each of rating values of the plurality of candidates, based on the determined competition result.

8. The suggestion device according to claim 7, wherein the calculator preferentially extracts, from among the plurality of candidates, a candidate that has not learned an estimation model for the candidate or a candidate having a small number of times of learning, and thus sets the extracted candidate as the calculation candidate.

9. The suggestion device according to claim 7, wherein, in the suggestion device,

the combination is expressed by an n-dimensional binary vector with respect to the number n of the plurality of parameters, and,
when a binary vector expressing a combination represented by the estimation candidate is input, the rating model outputs a rating value for the estimation candidate, and,
for a stage t = 1, 2, ..., and k in order, by using permutations $a_1$, $a_2$, ..., and $a_k$ in which the number of parameters related to a combination represented by the useful candidate is k or less and integers from 1 to k are arranged, a candidate in which the number of parameters related to a combination is t is extracted as the two or more candidates from the plurality of candidates, and is caused to compete in the competition processing, and, after the competition processing is repeated, the plurality of candidates are narrowed down by the estimator and the remover.

10. The suggestion device according to claim 1, wherein

the outputter outputs the useful candidate by drawing the useful candidate in a scatter diagram,
a first axis in the scatter diagram indicates the number of parameters related to a combination represented by the useful candidate, or a total cost for acquiring a parameter related to the combination, and
a second axis in the scatter diagram indicates performance or a rating value calculated for the useful candidate.

11. The suggestion device according to claim 1, wherein

the target is a patient,
the plurality of parameters is a plurality of examination items to be applied to the patient,
the plurality of parameter values is examination result values each acquired by applying the plurality of examination items to the patient,
the label identifies a prognosis of the patient, and
the factor parameter is a prognosis factor contributing to a prediction of a prognosis of the patient.

12. The suggestion device according to claim 6, wherein

the target is a cancer patient,
the plurality of parameters is a plurality of examination items to be applied to the cancer patient, and includes determination of a score or classification, based on a plurality of blood examinations to be applied to the cancer patient, and a medical photograph image,
the plurality of parameter values is examination result values each acquired by applying the plurality of examination items to the cancer patient,
the factor parameter is a prognosis factor contributing to a prediction of a prognosis of the cancer patient,
the image is a medical photograph image captured for the cancer patient, and
the label represents presence or absence of a recurrence in the cancer patient.

13. The suggestion device according to claim 6, wherein

the target is a patient suspected of cancer,
the plurality of parameters is a plurality of blood examinations to be applied to the patient,
the plurality of parameter values is examination result values each acquired by applying the plurality of examination items to the patient,
the factor parameter is a factor contributing to a prediction of whether the patient has a highly malignant tumor,
the image is an echo photograph image captured for the patient, and
a label in the plurality of records is a determination result of a score or classification based on a medical sample.

14. A suggestion system comprising:

an external device; and
the suggestion device according to claim 2, wherein
the external device makes designation of the parameter to be acquired to the suggestion device,
the suggestion device outputs the useful candidate to the external device, and
the external device

accepts a combination of already-acquired parameters for a target, and
presents, for the output useful candidate, a parameter (hereinafter referred to as an "unacquired parameter") not being the accepted already-acquired parameter among parameters related to a combination represented by the useful candidate.

15. The suggestion system according to claim 14, wherein
the external device further presents, for the output useful candidate, at least any one of

the number of the unacquired parameters,
an additional cost for acquiring the unacquired parameter, and
performance or a rating value calculated for the useful candidate.

16. The suggestion system according to claim 15, wherein

the external device further makes designation of a combination of the accepted already-acquired parameters to the suggestion device,
the suggestion device further outputs, to the external device, a candidate (hereinafter referred to as an "already-acquired candidate") having best performance or a best rating value being calculated for a calculation candidate representing a partial combination included in the designated combination, and performance or a rating value of the already-acquired candidate, and
the external device further presents the output performance or the output rating value for the already-acquired candidate.

17. The suggestion system according to claim 16, wherein

the suggestion device

outputs a plurality of combinations of the useful candidate and an estimation model learned for the useful candidate,
estimates a plurality of labels to be provided to the target by providing, as input data, a part or a whole of the transmitted parameter value to each estimation model of the plurality of combinations, and
transmits the plurality of estimated labels to the external device, and

the external device presents the plurality of transmitted labels.

18. The suggestion system according to claim 17, wherein

the external device is a terminal for a medical worker who deals with a patient,
the parameter is an examination item to be applied to the patient,
the already-acquired parameter is an examination item that has been applied to the patient,

the unacquired parameter is an examination item that has not been applied yet to the patient,

the parameter value is an examination value to be acquired by applying the examination item to the patient,

a prognosis of the patient is classified by the label,

the terminal presents an already-acquired examination value for the patient on a screen,

the suggestion device or the terminal selects, as a prediction candidate from the plurality of useful candidates, a useful candidate in which the already-acquired examination value fulfills a combination of examination items related to a useful candidate,

the suggestion device provides, as an input, the already-acquired examination value that fulfills a combination related to the selected prediction candidate to an estimation model learned for the selected prediction candidate, and causes the estimation model to estimate a prognosis of the patient, and

the terminal presents the estimated prognosis on the screen.

19. A suggestion system for a medical worker who deals with a patient, the suggestion system comprising:

an examination value presenter that presents an already-acquired examination value for the patient on a screen;

a prediction candidate selector that selects, as a prediction candidate, a useful candidate in which the already-acquired examination value fulfills a combination of examination items related to a useful candidate, from a plurality of useful candidates being a plurality of useful candidates and each representing a combination of examination items;

a prognosis estimator that provides, as an input, the already-acquired examination value that fulfills a combination related to the selected prediction candidate to an estimation model learned for the selected prediction candidate, and causes the estimation model to estimate a prognosis of the patient; and

a prognosis presenter that presents the estimated prognosis on the screen.

20. The suggestion system according to claim 19, wherein

each useful candidate of the plurality of useful candidates further represents performance of the estimation model learned for the each useful candidate,

the prediction candidate selector selects, as a recommendation candidate from the plurality of useful candidates, a useful candidate in which performance related to a useful candidate is better in terms of performance related to the selected prediction candidate, and

the suggestion system further comprises a recommendation presenter that presents, on the screen,

an examination item not allotted by the already-acquired examination value among combinations related to the selected recommendation candidate, and

contrast information that contrasts performance related to the selected recommendation candidate with performance related to the selected prediction candidate.

21. The suggestion system according to claim 19, wherein

the selected prediction candidate is plural, and

the suggestion system further comprises a similarity presenter that presents, on the screen, a degree of similarity or variations of a plurality of prognoses being estimated for the plurality of selected prediction candidates as related information being related to

reliability of a plurality of prognoses or a risk being estimated for the plurality of selected prediction candidates, and

reliability or abnormality of the already-acquired examination value.

22. The suggestion system according to claim 19, wherein

the examination value presenter further presents a photograph image captured for the patient, and

a feature parameter or a feature vector acquired by performing dimensionality compression on the photograph image is further provided as the input to the estimation model.

23. The suggestion system according to claim 22, wherein

the patient is a patient suspected of cancer,

the plurality of examination items is a plurality of blood examinations to be applied to the patient, and

the photograph image is a medical photograph image or an echo photograph image.

24. The suggestion system according to claim 23, wherein

the estimation model estimates two prognoses of a prognosis when a surgical or invasive operation is performed on the patient and a prognosis when the operation is not performed on the patient, and
the prognosis presenter presents at least any one of

the two estimated prognoses, and
whether there is a difference between the two estimated prognoses.

25. A suggestion method in which a suggestion device suggests, based on a plurality of records, any one or more candidates of a plurality of candidates each representing a combination of parameters to be acquired for a target, as a useful candidate representing a combination of factor parameters contributing to a decision of a label provided to the target, each record of the plurality of records including a plurality of parameter values each acquired by acquiring a plurality of parameters for a target associated with the each record, and a label provided to the associated target, the suggestion method comprising:

a calculation step of

learning, by referring to the plurality of records, an estimation model for each calculation candidate of a calculation candidate being a part of the plurality of candidates,
calculating, by the learned estimation model, performance that estimates a label provided to a target from a parameter value in which a parameter related to a combination represented by the each calculation candidate is acquired for the target, and
calculating a rating value associated with the each calculation candidate, based on the calculated performance;

an estimation step of

learning a rating model by referring to a rating value calculated for the calculation candidate, and
estimating a rating value associated with each estimation candidate of an estimation candidate other than the calculation candidate among the plurality of candidates, based on the learned rating model; and

an output step of outputting, as the useful candidate, a calculation candidate associated with a rating value being high-ranking among the calculated or estimated rating values.

26. A program causing a computer to suggest, based on a plurality of records, any one or more candidates of a plurality of candidates each representing a combination of parameters to be acquired for a target, as a useful candidate representing a combination of factor parameters contributing to a decision of a label provided to the target, each record of the plurality of records including a plurality of parameter values each acquired by acquiring a plurality of parameters for a target associated with the each record, and a label provided to the associated target, the program causing the computer to function as:

a calculator that

learns, by referring to the plurality of records, an estimation model for each calculation candidate of a calculation candidate being a part of the plurality of candidates,
calculates, by the learned estimation model, performance that estimates a label provided to a target from a parameter value in which a parameter related to a combination represented by the each calculation candidate is acquired for the target, and
calculates a rating value associated with the each calculation candidate, based on the calculated performance;

an estimator that

learns a rating model by referring to a rating value calculated for the calculation candidate, and
estimates a rating value associated with each estimation candidate of an estimation candidate other than the calculation candidate among the plurality of candidates, based on the learned rating model; and

an outputter that outputs, as the useful candidate, a calculation candidate associated with a rating value being high-ranking among the calculated or estimated rating values.

27. A non-transitory computer-readable information recording medium recording the program according to claim 26.

# FIG. 1

101

122

PLURAL
CANDIDATES

121

CALCULATOR  102

PLURAL
RECORDS

RATING VALUE OF
CALCULATION
CANDIDATE

105

REMOVER

ESTIMATOR  103

RATING VALUE OF
ESTIMATION CANDIDATE

104

OUTPUTTER

USEFUL CANDIDATE

# FIG. 2

input_1: InputLayer

↓

dense_1: Dense

↓

dropout_1: Dropout

↓

dense_2: Dense

↓

dropout_2: Dropout

↓

dense_3: Dense

↓

dropout_3: Dropout

↓

dense_4: Dense

↓

dropout_4: Dropout

↓

dense_5: Dense

# FIG. 3

```
┌──────────────────┐
│   SUGGESTION     │
│   PROCESSING     │
└──────────────────┘
         │                              S221
         │              ┌──────────────────┐              S231
      S201              │  LEARN RATING    │      ┌──────────────────┐
┌──────────────────┐   │     MODEL        │      │  OUTPUT USEFUL   │
│   INITIALIZE     │   └──────────────────┘      │   CANDIDATE      │
└──────────────────┘         │    S222            └──────────────────┘
      S202              ┌──────────────────┐              │
┌──────────────────┐   │  FOR REMAINING   │      ┌──────────────────┐
│ GENERATE PLURAL  │   │ESTIMATION CANDIDATE│    │      END         │
│   CANDIDATES     │   └──────────────────┘      └──────────────────┘
└──────────────────┘         │    S223
      S203  Yes        ┌──────────────────┐
┌──────────────────┐   │  ESTIMATE RATING │
│   HIGH-RANKING   │   │     VALUE        │
│    CONDITION     │   └──────────────────┘
│  IS SATISFIED?   │         │    S224
└──────────────────┘   ┌──────────────────┐
       No  S204        │  UPDATE RATING   │
┌──────────────────┐   │     VALUE        │
│   CALCULATION    │   └──────────────────┘
│COMPLETION CONDITION│       │    S225
│  IS SATISFIED?   │   ┌──────────────────┐
└──────────────────┘   │     REPEAT       │
       No  S205   Yes  └──────────────────┘
┌──────────────────┐         │    S226
│EXTRACT CALCULATION│  ┌──────────────────┐
│   CANDIDATE      │   │  ACQUIRE HIGH-   │
└──────────────────┘   │ RANKING CANDIDATE│
      S206             └──────────────────┘
┌──────────────────┐              S208
│REPEAT FOR NUMBER OF│  ┌──────────────────┐
│TIMES OF COMPETITIONS│ │  FOR EXTRACTED   │
└──────────────────┘   │CALCULATION CANDIDATE│
      S207             └──────────────────┘
┌──────────────────┐         │    S209
│GENERATE TRAINING RECORD│┌──────────────────┐
│  AND TEST RECORD │   │ GENERATE TRAINING│
└──────────────────┘   │  DATA AND TEST DATA│
      S213             └──────────────────┘
┌──────────────────┐         │    S210
│DETERMINE WINNING AND│  ┌──────────────────┐
│LOSING OF COMPETITION││ │ LEARN ESTIMATION │
└──────────────────┘   │     MODEL        │
      S214             └──────────────────┘
┌──────────────────┐         │    S211
│     REPEAT       │   ┌──────────────────┐
└──────────────────┘   │   CALCULATE      │
      S215             │  PERFORMANCE     │
┌──────────────────┐   └──────────────────┘
│UPDATE RATING VALUE│        │    S212
│  OF CALCULATION  │   ┌──────────────────┐
│   CANDIDATE      │   │     REPEAT       │
└──────────────────┘   └──────────────────┘
```

# FIG. 4

```
    ┌─────────────────┐
    │   SUGGESTION    │
    │   PROCESSING    │
    └─────────────────┘
             │  S201
    ┌─────────────────┐
    │   INITIALIZE    │
    └─────────────────┘
             │  S202
    ┌─────────────────┐
    │ GENERATE PLURAL │
    │   CANDIDATES    │
    └─────────────────┘
             │  S301
    ╱─────────────────╲
    │  t = 1, 2, ···, k │
    ╲─────────────────╱
             │  S302
    ╱─────────────────╲
    │ REPEAT FOR NUMBER │
    │     OF TIMES      │
    │  ACCORDING TO t   │
    └─────────────────┘
             │  S303
    ┌─────────────────┐
    │ CALCULATE AND UPDATE │
    │    RATING VALUE BY    │
    │   CAUSING CANDIDATE   │
    │   HAVING PARAMETER    │
    │ NUMBER t TO COMPETE   │
    └─────────────────┘
             │  S304
    ┌─────────────────┐
    │ REMOVE CANDIDATE  │
    │ HAVING PARAMETER  │
    │ NUMBER t AND LOW- │
    │  RANKING RATING   │
    │      VALUE        │
    └─────────────────┘
             │  S305
    ╱─────────────────╲
    │     REPEAT        │
    └─────────────────┘
             │  S306
    ┌─────────────────┐
    │  SELECT CANDIDATE  │
    │ HAVING HIGH-RANKING │
    │ RATING VALUE AS HIGH-│
    │RANKING CANDIDATE FROM│
    │  CANDIDATES HAVING   │
    │  PARAMETER NUMBER t  │
    └─────────────────┘
             │
    ╱─────────────────╲
    │     REPEAT        │
    └─────────────────┘
             │  S307
```

```
             S308
    ┌─────────────────┐
    │ CALCULATE RATING │
    │ VALUE OF HIGH-   │
    │ RANKING CANDIDATE│
    │ BY ROUND-ROBIN   │
    │ COMPETITION      │
    └─────────────────┘
             │  S309
    ┌─────────────────┐
    │ OUTPUT HIGH-     │
    │ RANKING CANDIDATE│
    │ HAVING HIGH-     │
    │ RANKING RATTING  │
    │ VALUE AS USEFUL  │
    │ CANDIDATE        │
    └─────────────────┘
             │
    ┌─────────────────┐
    │       END       │
    └─────────────────┘
```

FIG. 5

EP 4 597 514 A1

## FIG. 6

SCATTER DIAGRAM

COST

- 175 — ● RF_echo_PSA_WBC_Hb_GPT_cre
- 170
- ● RF_echo_PSA_WBC_Hb_Alb_Ca
- 165 — RF_echo_PSA_Hb_GOT
- ● ● RF_echo_PSA_WBC_Hb_CRP
- 160
- ● ● RF_echo_PSA_WBC_Hb_Alb
- 155 — RF_echo_PSA_Hb_cre
- 150
- 145 — ● RF_echo_PSA_WBC / RF_echo_PSA_Hb / RF_echo_PSA_WBC_Hb
- RF_echo_PSA_WBC_Hb_Plt
- 140

RATING: 1450  1500  1550  1600  1650

# FIG. 7

EP 4 597 514 A1

| COMBINATION NAME | RATING |
|---|---|
| RF_echo_PSA_WBC_Hb | 1635.710833 |
| RF_echo_PSA_WBC | 1578.544444 |
| RF_echo_PSA_Hb | 1575.473352 |
| RF_echo_PSA_WBC_Hb_Alb_Ca | 1532.266206 |
| RF_echo_PSA_WBC_Hb_CRP | 1520.272607 |
| RF_echo_PSA_WBC_Hb_Alb | 1516.125097 |
| RF_echo_PSA_Hb_cre | 1509.518065 |
| RF_echo_PSA_WBC_Hb_Plt | 1507.569392 |
| RF_echo_PSA_Hb_GOT | 1502.329218 |
| RF_echo_PSA_WBC_Hb_GPT_cre | 1497.454263 |

M E N U

155

● RF_echo_PSA_WBC_Hb_GPT_cre

● RF_echo_PSA_WBC_Hb_Alb_Ca

SCATTER DIAGRAM

_PSA_Hb_GO

RF_echo_PSA_Hb_

| COMBINATION NAME | AUC (PROGNOSIS PREDICTION AFTER 1 YEAR) |
|---|---|
| RF_echo_PSA_WBC_Hb | 0.828 |
| RF_echo_PSA | 0.795 |
| RF_echo_Hb | 0.687 |
| RF_echo_WBC | 0.622 |

| COMBINATION NAME | % OF BEING INCLUDED IN HIGH-RANKING COMBINATION |
|---|---|
| echo_PSA | 84% |
| echo_Hb | 72% |
| echo_PSA_Hb | 64% |
| echo_PSA_WBC | 48% |
| echo_PSA_WBC_Hb | 44% |

140

REGION OF HIGH
COST PERFORMANCE

RF_echo_PSA_WBC

RF_echo_PSA_Hb   RF_echo_PSA_WBC_Hb

PSA_WBC_Hb_Plt

1450   1500   1550   1600   1650

RATING

# FIG. 8

501

502

BIG DATA SERVER

101

SUGGESTION
DEVICE

504

COMPUTER
COMMUNICATION
NETWORK

503

EXTERNAL
DEVICE

503

EXTERNAL
DEVICE

503

EXTERNAL
DEVICE

# FIG. 9

EXTERNAL DEVICE

BIG DATA SERVER

503

502

DESIGNATION OF ACQUIRABLE PARAMETER AND LABEL CLASSIFICATION (601)

SUGGESTION PROCESSING (602)

USEFUL CANDIDATE (603)

USEFUL CANDIDATE (604)

# FIG. 10

EXTERNAL DEVICE

BIG DATA
SERVER

503

502

TRANSMIT DESIGNATION OF
ACQUIRABLE PARAMETER AND
LABEL CLASSIFICATION,
ALREADY-ACQUIRED
PARAMETER, AND IMAGE
(701)

SUGGESTION PROCESSING
(702)

EXTRACT APPLICABLE
CALCULATION
CANDIDATE (703)

SELECT ALREADY-
ACQUIRED
CANDIDATE (704)

USEFUL CANDIDATE AND
ALREADY-ACQUIRED CANDIDATE
(705)

PERFORMANCE AND THE LIKE
OF USEFUL CANDIDATE AND
ALREADY-ACQUIRED
CANDIDATE (706)

ESTIMATE
LABEL
(707)

LABEL (708)

LABEL (709)

# FIG. 11

801

SPECIFICATION OF PATIENT,
ALREADY-ACQUIRED EXAMINATION VALUE,
PHOTOGRAPH

911

802

SCREEN ← EXAMINATION
VALUE PRESENTER

803

PREDICTION
CANDIDATE
SELECTOR ← USEFUL CANDIDATE

PREDICTION
CANDIDATE

804

PROGNOSIS
ESTIMATOR

ESTIMATION
MODEL

805

PROGNOSIS
PRESENTER ← ESTIMATED
PROGNOSIS

807

SIMILARITY
PRESENTER

806

RECOMMENDATION
PRESENTER

PROGNOSIS INFORMATION

RELATED INFORMATION

RECOMMENDATION INFORMATION

# FIG. 12

ELECTRONIC MEDICAL CHART — 901, 902, 906

| EXAM ITEM | EXAM VALUE | EXAM DATE |
|---|---|---|
| PSA | 6.2ng/mL | 2023-09-22 |
| Hb | 18.2g/dL | 2023-09-22 |
| WBC | ——— | ——— |

PATIENT ID/NAME: 1234 XXX YYYY

IMAGE
2023-09-22 12:34:56
ECHO PHOTOGRAPH

✓ RECURRENCE OF PROSTATE CANCER AFTER 1 YEAR
RECURRENCE OF PROSTATE CANCER AFTER 3 YEARS
RECURRENCE OF PROSTATE CANCER AFTER 5 YEARS

MAKE PROGNOSIS PREDICTION

904  905  903  908  909

# FIG. 13

EP 4 597 514 A1

**ELECTRONIC MEDICAL CHART**  ☒ (901, 902, 931)

PATIENT ID/NAME  **1234 XXX YYYY**  IMAGE

| EXAM ITEM | EXAM VALUE | EXAM DATE |
|---|---|---|

2023-09-22 12:34:56

PROGNOSIS PREDICTION OF RECURRENCE OF PROSTATE CANCER AFTER 1 YEAR  ☒

RESULT OF PROGNOSIS PREDICTION IS

PS

Hb

WB

PRINT   TRANSCRIBE TO CHART

MAKE PROGNOSIS PREDICTION (909)

904  905  903  932

# FIG. 14

```
        ┌─────────────────┐
        │   PREDICTION    │
        │   PROCESSING    │
        └────────┬────────┘
                 │  S951
        ┌────────┴────────┐
        │DISPLAY ELECTRONIC│
        │  MEDICAL CHART  │
        └────────┬────────┘
                 │  S952
        ┌────────┴────────┐
        │ ALLOW SELECTION OF│
        │DESIRED PREDICTION│
        └────────┬────────┘
                 │  S953
        ┌────────┴────────┐
        │ ACQUIRE USEFUL  │
        │   CANDIDATE     │
        └────────┬────────┘
                 │  S954
        ┌────────┴────────┐
        │SELECT PREDICTION│
        │   CANDIDATE     │
        └────────┬────────┘
                 │  S955
        ┌────────┴────────┐
        │    FOR EACH      │
        │PREDICTION CANDIDATE│
        └────────┬────────┘
                 │  S956
        ┌────────┴────────┐
        │ESTIMATE PROGNOSIS│
        └────────┬────────┘
                 │  S957
        ┌────────┴────────┐
        │     REPEAT       │
        └────────┬────────┘
                 │  S958
        ┌────────┴────────┐
        │GENERATE ADVICE BASED ON│
        │ESTIMATED PROGNOSIS│
        └────────┬────────┘
```

S959 — THERE IS RECOMMENDATION CANDIDATE? — No

Yes — S960 ACQUIRE EXAMINATION ITEM TO BE ADDED

S961 — ADD RECOMMENDATION INFORMATION TO ADVICE

S962 — PRESENT ADVICE

END

## INTERNATIONAL SEARCH REPORT

| International application No. |
|---|
| **PCT/JP2023/035267** |

**A. CLASSIFICATION OF SUBJECT MATTER**

**G16H 50/00**(2018.01)i; **G06N 20/00**(2019.01)i; **G16H 10/40**(2018.01)i
FI:    G16H50/00; G06N20/00 130; G16H10/40

According to International Patent Classification (IPC) or to both national classification and IPC

**B. FIELDS SEARCHED**

Minimum documentation searched (classification system followed by classification symbols)

G16H10/00-80/00; G06N20/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2019-121390 A (CANON MEDICAL SYSTEMS CORP.) 22 July 2019 (2019-07-22) | 1-27 |
| A | JP 2006-511880 A (GE MEDICAL SYSTEMS GLOBAL TECHNOLOGY COMPANY, LLC) 06 April 2006 (2006-04-06) | 1-27 |

☐ Further documents are listed in the continuation of Box C.    ☑ See patent family annex.

| | |
|---|---|
| \*    Special categories of cited documents: | "T" later document published after the international filing date or priority date and not in conflict with the application but cited to understand the principle or theory underlying the invention |
| "A"  document defining the general state of the art which is not considered to be of particular relevance | |
| "E"  earlier application or patent but published on or after the international filing date | "X"  document of particular relevance; the claimed invention cannot be considered novel or cannot be considered to involve an inventive step when the document is taken alone |
| "L"  document which may throw doubts on priority claim(s) or which is cited to establish the publication date of another citation or other special reason (as specified) | "Y"  document of particular relevance; the claimed invention cannot be considered to involve an inventive step when the document is combined with one or more other such documents, such combination being obvious to a person skilled in the art |
| "O"  document referring to an oral disclosure, use, exhibition or other means | |
| "P"  document published prior to the international filing date but later than the priority date claimed | "&"  document member of the same patent family |

| Date of the actual completion of the international search | Date of mailing of the international search report |
|---|---|
| **15 November 2023** | **28 November 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT
### Information on patent family members

International application No.

**PCT/JP2023/035267**

| Patent document cited in search report | Publication date (day/month/year) | Patent family member(s) | Publication date (day/month/year) |
|---|---|---|---|
| JP 2019-121390 A | 22 July 2019 | US 2019/0214138 A1 | |
| JP 2006-511880 A | 06 April 2006 | US 2004/0122708 A1 | |
| | | WO 2004/061742 A2 | |
| | | EP 1576527 A2 | |
| | | KR 10-2005-0084394 A | |
| | | CN 1751309 A | |
| | | AU 2003297268 A1 | |

Form PCT/ISA/210 (patent family annex) (January 2015)

**EP 4 597 514 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6945253 B
- US 8538910 B
- JP 2022155487 A
- JP 2023086586 A